# FASCICULE DE BREVET EUROPEEN

(11) **EP 1 642 893 B1**
(45) Date de publication et mention de la délivrance du brevet: **25.05.2011**
(21) Numéro de dépôt: 05291622.8
(22) Date de dépôt: 29.07.2005
(51) Int. Cl.: C07D 251/54, C07D 251/70, A61Q 1/00, A61Q 17/00

(54) **Composés s-triazine possédant deux substituants choisis parmi des groupes para-aminobenzalmalonate et para-aminobenzalmalonamide encombrés et un substituant aminobenzoate, compositions, utilisations**
S-Triazinverbindungen besitzend zwei Substituenten ausgewählt aus der Gruppe von sperrigen para-Aminobenzalmalonat- und para-Aminobenzalmalonamidgruppen und einem Aminobenzoatesubstituenten, deren Zusammensetzungen und Verwendungen.
s-triazine compounds possessing two substituents selected from bulky para-aminobenzalmalonate and para-aminobenzalmalonamide groups and one aminobenzoate substituent, compositions and uses thereof

(30) Priorité: 02.09.2004 FR 0409292
(43) Date de publication de la demande: 05.04.2006
(73) Titulaire: L'Oréal, 75008 Paris (FR)
(72) Inventeur: Richard, Hervé, 93420 Villepinte (FR); Lejuste, Catherine, 78110 Le Vésinnet (FR)
(74) Mandataire: Dodin, Catherine

(56) Documents cités:
- EP-A- 0 507 691
- EP-A- 0 841 341
- EP-A- 1 269 980

## Description

L'invention concerne de nouveaux composés de s-triazine possédant deux substituants choisis parmi des groupes para-aminobenzalmalonate et para-aminobenzalmalonamide encombrés et un substituant aminobenzoate ou aminobenzamide et leurs utilisations en cosmétique à titre de filtres solaires actifs dans le domaine des rayonnements UV.

L'invention concerne également des compositions photoprotectrices contenant ces composés s-triazine à titre de filtres solaires actifs dans le domaine des rayonnements UV.

On sait que les radiations de longueurs d'onde comprises entre 280 nm et 400 nm permettent le brunissement de l'épiderme humain, et que les radiations de longueurs d'onde comprises entre 280 et 320 nm connues sous la dénomination de radiations UV-B, provoquent des érythèmes et des brûlures cutanées qui peuvent nuire au développement du bronzage naturel.

On sait également que les rayons UV-A, de longueurs d'onde comprises entre 320 et 400 nm, provoquant le brunissement de la peau, sont susceptibles d'induire une altération de celle-ci, notamment dans le cas d'une peau sensible et/ou continuellement exposée au rayonnement solaire. Les rayons UV-A entraînent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photoallergiques. Ainsi, pour des raisons esthétiques et cosmétiques telles que la conservation de l'élasticité naturelle de la peau, de plus en plus de personnes désirent contrôler l'effet des rayons UV-A sur leur peau. On entend par facteur de protection solaire le rapport du temps d'irradiation nécessaire pour atteindre le seuil érythématogène en présence du filtre testé au temps d'irradiation nécessaire pour atteindre ce même seuil en l'absence de filtre.

Il est donc souhaitable de disposer de composés susceptibles d'absorber toute la gamme des UV, à la fois les rayons UV-B et les UV-A.

Outre leur pouvoir filtrant des rayonnements UV-B et UV-A, les composés photoprotecteurs recherchés doivent également présenter de bonnes propriétés cosmétiques, une bonne solubilité dans les solvants usuels et notamment dans les corps gras tels que les huiles et les graisses, ainsi qu'une bonne résistance à l'eau et à la transpiration (rémanence) et une très bonne photostabilité.

Parmi tous les composés qui ont été préconisés à cet effet, on peut citer notamment les dérivés de s-triazine portant des substituants para-aminobenzalmalonates décrits dans les demandes EP 0 507 691 et EP 0 841 341 de la Demanderesse. Ces composés possèdent cependant une stabilité photochimique et une solubilité dans les solvants usuels qui n'est pas encore pleinement satisfaisante.

La demanderesse a découvert de manière surprenante une nouvelle famille de dérivés de s-triazine possédant deux substituants choisis parmi des groupes para-aminobenzalmalonate et para-aminobenzalmalonamide encombrés et un substituant aminobenzoate ou aminobenzamide ayant de bonnes propriétés absorbantes dans la gamme totale des rayons UV-A ainsi q'une contribution importante dans le domaine des UVB et une photostabilité et une solubilité nettement améliorée par rapport aux dérivés de s-triazine greffés par des para-aminobenzatmatonates de l'art antérieur évoqués précédemment.

L'invention concerne une nouvelle famille de dérivés de s-triazine possédant deux substituants choisis parmi des groupes para-aminobenzalmalonate et para-aminobenzalmalonamide encombrés et un substituant aminobenzoate ou aminobenzamide contenus dans la formule (I) que l'on définira plus loin en détail.

L'invention concerne également une composition cosmétique ou dermatologique, destinée à la photoprotection des matières kératiniques, contenant dans un milieu physiologiquement acceptable au moins un composé de formule (I).

On entend par «milieu physiologiquement acceptable», un milieu non toxique et susceptible d'être appliqué sur la peau, les lèvres, les cheveux, les cils, les sourcils les ongles. La composition de l'invention peut constituer notamment une composition cosmétique ou dermatologique.

D'autres objets apparaîtront à la lumière de la description.

Les composés conformes à la présente invention répondent à la formule générale (I) suivante : 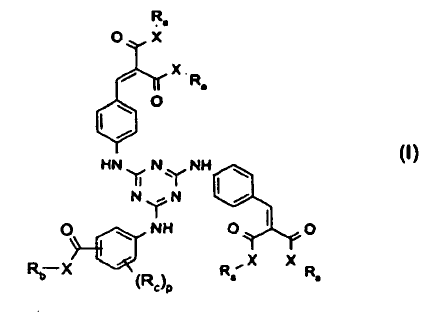 dans laquelle :
X, identiques ou différents, désignent -0- ou -NR₆-
Rₐ, identiques ou différents, désignent un groupe de formule (II) : 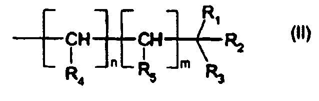 dans laquelle :
   R₁ et R₂, identiques ou différents représentent un groupe alkyle en C₁-C₈, linéaire ou ramifié, .
   R₁ et R₂ peuvent former un cycle en C₅-C₈, éventuellement substitué par 1, 2 ou 3 groupements alkyle(s) en C₁-C₄, linéaire(s) ou ramifié(s) ;
   R₃, R₄ et R₅, identiques ou différents représentent un atome d'hydrogène ou un groupe alkyle en C₁-C₄, linéaire ou ramifié ;
   n vaut 0 ou 1 ;
   m vaut 0 ou 1 ;
   sous réserve que :
      (i) lorsque n = 1 et R₄ désigne l'hydrogène alors m est égal à 0 et ,
      (ii) lorsque R₁ et R₂ forment un cycle en C₅-C₈ alors la somme n+m est différente de 2 ;
   R₆ représente l'hydrogène ou un groupe alkyle en C₁-C₈,
R_{b} désigne un groupe alkyle en C₁-C₂₀, linéaire ou ramifié et éventuellement insaturé, un groupe cycloalkyle en C₅-C₁₂, éventuellement substitué par 1 à 3 radicaux alkyles en C₁-C₄, linéaires ou ramifiés, le groupe -(CH₂CHR₇-O)_{q}R₈ ou le groupe -CH₂-CH(OH)-CH₂-O-R₈,
R₇ représente l'hydrogène ou méthyle,
R₈ représente l'hydrogène ou un groupe alkyle en C₁-C₈,
q = 1-20,
le groupement COXR_{b} peut être en position ortho, méta ou para du groupement amino, R_{c} désigne un radical alkyle en C₁-C₂₀, linéaire ou ramifié, saturé ou insaturé, le radical OH, un radical alcoxy en C₁-C₂₀, linéaire ou ramifié,
p est égal à 0, 1 ou 2
et sont caractérisés par le fait qu'ils sont choisis parmi :
(1) ceux pour lesquels les deux conditions suivantes sont réunies :
   (a) n=m=0 et
   (b) R₁, R₂, R₃ désignent un alkyle en C₁-C₄.
   ou bien R₃ désigne hydrogène et R₁ et R₂ forment un cycle en C5-C8 éventuellement substitué par 1 ou deux radicaux alkyle ;
(2) ceux pour lesquels les deux conditions suivantes sont réunies :
   (a) n=1 et R₄ désigne un alkyle en particulier méthyle ou m = 1 et R₅ désigne un alkyle en particulier méthyle et
   (b) R₁ et R₂ désignent un alkyle en C₁-C₄ et plus particulièrement méthyle ;
(3) les composés de formule (1), (2), (3) (4), (5), (9) ou (10) que l'on définira ci-dessous ;
(4) le composé de formule (6) que l'on définira ci-dessous.

Dans la formule (I) ci-dessus, les radicaux alkyles peuvent être choisis notamment au sein des radicaux méthyle, éthyle, n-propyle, isopropyle, n-butyle, isobutyle, tert-butyle, n-amyle, isoamyle, néopentyle, n-hexyle, n-heptyle, n-octyle, éthyl-2 hexyle et tert-octyle. Le radical alkyle particulièrement préféré est le radical méthyle.

Les radicaux cycloalkyles peuvent être choisis notamment au sein des radicaux cyclopentyle, cyclohexyle et cycloheptyle. Le radical cycloalkyle particulièrement préféré est le radical cyclohexyle. Ces radicaux peuvent être substitués par des radicaux alkyle en C₁-C₄ choisis de préférence parmi méthyle, isopropyle et tert-butyle.

Parmi les composés de formule (I) préférés, on citera ceux pour lesquels les deux conditions suivantes sont réunies :
(a) n=m=0 et
(b) R₁, R₂, R₃ désignent méthyle ou bien R₃ désigne hydrogène et R₁ et R₂ forment un cycle en C₅-C₈ éventuellement substitué par 1 ou deux radicaux alkyle et plus particulièrement cyclohexyle.

Parmi les composés de formule (I) préférés, on citera également ceux pour lesquels les deux conditions suivantes sont réunies :
(a) n=1 et R₄ désigne méthyle ou m = 1 et R₅ désigne méthyle et
(b) R₁ et R₂ désignent méthyle.

Parmi les composés de formule (I) plus particulièrement préférés, on citera ceux choisis parmi les composés de formules (1) à (10) suivantes :

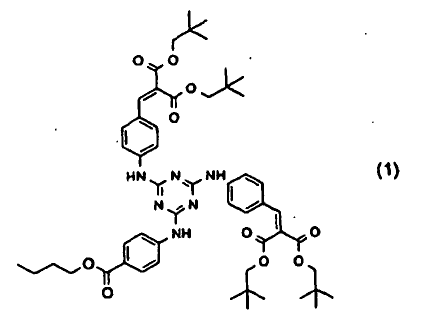

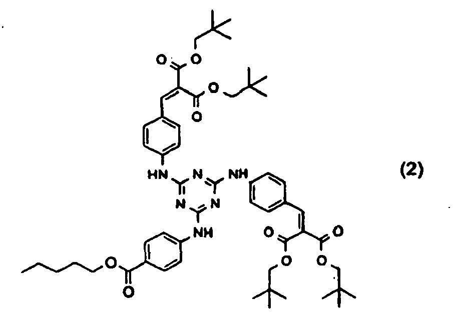

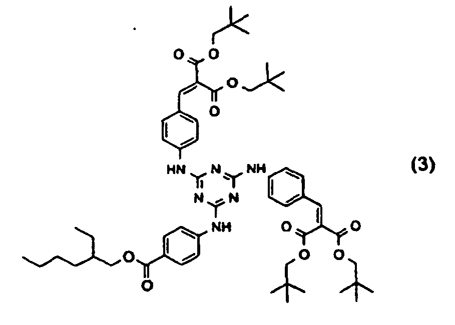

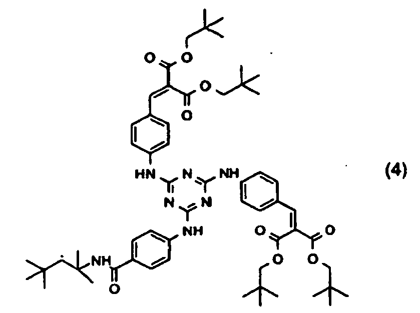

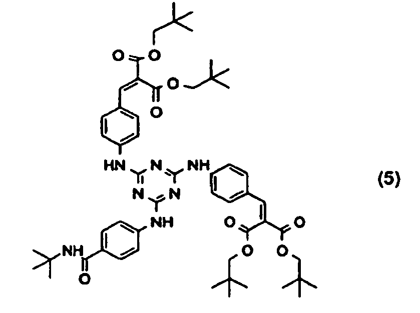

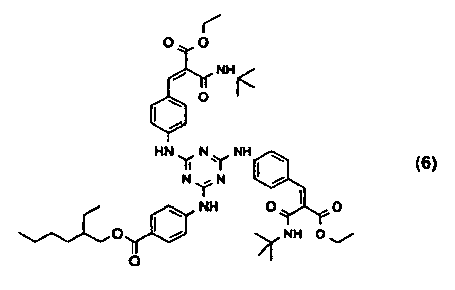

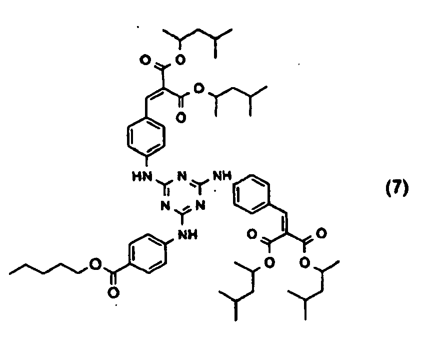

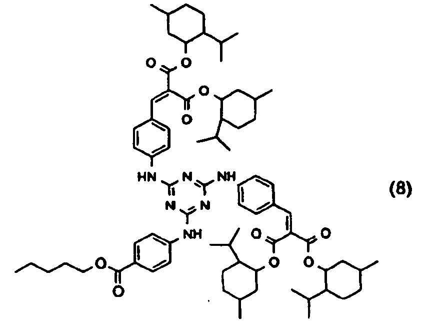

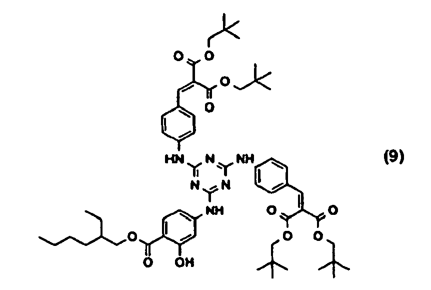

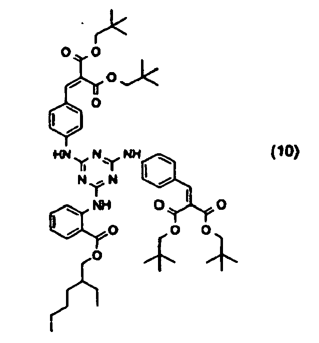

Parmi ces composés, on utilisera plus particulièrement le 2,4-bis-(4'-amino benzalmalonate de di-néopentyle)-6-(4"-amino benzoate de butyle)-s-triazine de formule (1).

Les dérivés de formule (I) peuvent être obtenus selon le schéma (A) suivant : 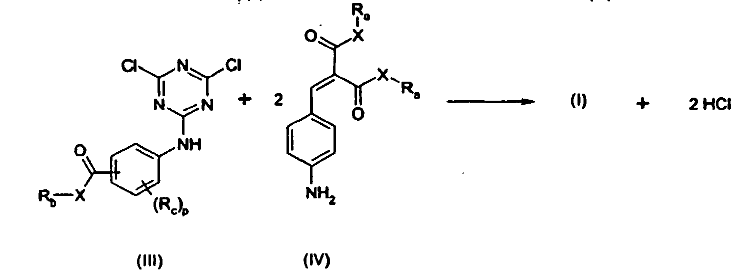 dans lequel Rₐ, X, R_{b}, R_{c} et p ont la définition de la formule (I) ci-dessus.

Les réactions ci-dessus peuvent être effectuées éventuellement en présence d'un solvant (par exemple : toluène, xylène ou dichloro-1,2-éthane), à une température comprise entre 0°C et 250°C, plus particulièrement entre 5°C et 150°C. Elles peuvent être également réalisées en microondes en présence ou non d'un solvant (par exemple : toluène, xylène ou dichloro-1,2-éthane) ou en présence ou non de 10% de graphite, à une température de 50 à 150°C, à une puissance de 50-150 Watts pendant une durée de 10 à 30 minutes.

Les composés de formule (III) peuvent être préparés selon des méthodes connues décrites par exemple dans la demande EP 0 507 691 de la Demanderesse.

L'invention concerne également une composition cosmétique ou dermatologique, contenant dans un milieu physiologiquement acceptable au moins un composé de formule (I).

Les composés de formule (I) sont généralement présents dans la composition de l'invention dans des proportions comprises entre 0,01 % et 20 % en poids, de préférence entre 0,1 % et 10 % en poids, par rapport au poids total de la composition.

Les compositions conformes à l'invention peuvent comporter en plus d'autres filtres UV complémentaires actifs dans l'UVA et/ou l'UVB. Les agents photoprotecteurs additionnels sont généralement présents dans les compositions selon l'invention dans des proportions allant de 0,01 à 20% en poids par rapport au poids total de la composition, et de préférence allant de 0,1 à 10% en poids par rapport au poids total de la composition.

Les compositions selon l'invention peuvent également contenir des agents de bronzage et/ou de brunissage artificiels de la peau (agents autobronzants), et plus particulièrement la dihydroxyacétone (DHA). Ils sont présents de préférence dans des quantité allant 0,1 à 10% en poids par rapport au poids total de la composition.

Les compositions conformes à la présente invention peuvent comprendre en outre des adjuvants cosmétiques classiques notamment choisis parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants ou tout autre ingrédient habituellement utilisé dans le domaine cosmétique et/ou dermatologique.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société WITCO, le palmitate d'octyle, le lanolate d'isopropyle, les triglycérides dont ceux des acides caprique/caprylique), les esters et éthers gras oxyéthylénés ou oxypropylénés; les huiles siliconées (cyclométhicone, polydiméthysiloxanes ou PDMS) ou fluorées, les polyalkylènes.

Comme composés cireux, on peut citer la paraffine, la cire de camauba, la cire d'abeille, l'huile de ricin hydrogénée.

Parmi les solvants organiques, on peut citer les alcools et polyols inférieurs. Ces derniers peuvent être choisis parmi les glycols et les éthers de glycol comme l'éthylène glycol, le propylène glycol, le butylène glycol, le dipropylène glycol ou le diéthylène glycol.

Comme épaississants hydrophiles, on peut citer les polymères carboxyvinyliques tels que les carbopols (carbomers) et les Pemulen (Copolymère acrylate/C10-C30-alkylacrylate) ; les polyacrylamides comme par exemple les copolymères réticulés vendus sous les noms Sepigel 305 (nom C.T.F.A. : polyacrylamide/C13-14 isoparaffin/Laureth 7) ou Simulgel 600 (nom C.T.F.A. : acrylamide / sodium acryloyldimethyltaurate copolymer /isohexadecane / polysorbate 80) par la société Seppic ; les polymères et copolymères d'acide 2-acrylamido 2-méthylpropane sulfonique, éventuellement réticulés et/ou neutralisés, comme le poly(acide 2-acrylamido 2-méthylpropane suifonique) commercialisé par la société Hoechst sous la dénomination commerciale « Hostacerin AMPS » (nom CTFA : ammonium polyacryldimethyltauramide) ; les dérivés cellulosiques tels que l'hydroxyéthylcellulose ; les polysaccharides et notamment les gommes telles que la gomme de xanthane ; et leurs mélangés.

Comme épaississants lipophiles, on peut citer les argiles modifiées telles que le l'hectorite et ses dérivés, comme les produits commercialisés sous les noms de Bentone.

Parmi les actifs, on peut citer :
- les agents anti-pollution et/ou agent anti-radicalaire ;
- les agents dépigmentants et/ou des agents pro-pigmentants ;
- les agents anti-glycation ;
- les inhibiteurs de NO-synthase ;
- les agents stimulant la synthèse de macromolécules dermiques ou épidermiques et/ou empêchant leur dégradation ;
- les agents stimulant la prolifération des fibroblastes ;
- les agents stimulant la prolifération des kératinocytes ;
- les agents myorelaxants ;
- les agents tenseurs.
- les agents desquamants ;
- les agents hydratants ;
- les agents anti-inflammatoires ;
- les agents agissant sur le métabolisme énergétique des cellules.
- les agents répulsifs contre les insectes
- les antagonistes de substances P ou de CRGP.

Bien entendu, l'homme de l'art veillera à choisir le ou les éventuels composés complémentaires cités ci-dessus et/ou leurs quantités de manière telle que les propriétés avantageuses attachées intrinsèquement aux compositions conformes à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile. Elles peuvent se présenter en particulier sous forme d'émulsion, simple ou complexe (H/E, E/H, H/E/H ou E/H/E) telle qu'une crème, un lait, ou sous la forme d'un gel ou d'un gel crème, sous la forme d'une lotion, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau ou eau-dans huile.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous la dénomination « DC 5225 C » par la société Dow Coming, et les alkyl-dimethicone copolyols tels que le Laurylmethicone copolyol vendu sous la dénomination "Dow Coming 5200 Formulation Aid" par la société Dow Coming ; le Cetyl dimethicone copolyol tel que le produit vendu sous la dénomination Abil EM 90R par la société Goldschmidt et le mélange de cétyl diméthicone copolyol, d'isostearate de polyglycérole (4 moles) et de laurate d'hexyle vendu sous la dénomination ABIL WE 09 par la société Goldschmidt. On peut y ajouter aussi un ou plusieurs co-émulsionnants, qui, de manière avantageuse, peuvent être choisis dans le groupe comprenant les esters alkylés de polyol. Comme esters alkylés de polyol, on peut citer notamment les esters de glycérol et/ou de sorbitan et par exemple l'isostéarate de polyglycérol, tel que le produit commercialisé sous la dénomination isolan Gl 34 par la société Goldschmidt, l'isostéarate de sorbitan, tel que le produit commercialisé sous la dénomination Arlacel 987 par la société ICI, l'isostéarate de sorbitan et le glycérol, tel que le produit commercialisé sous la dénomination Ariacel 986 par la société ICI, et leurs mélanges.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; les éthers d'alcool gras et de sucre, notamment les alkylpolyglucosides (APG) tels que le décylglucoside et le laurylglucoside commercialisés par exemple par la société Henkel sous les dénominations respectives Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tegocare CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidylglucoside commercialisé sous la dénomination Montanov 202 par la société Seppic. Selon un mode particulier de réalisation de l'invention, le mélange de l'alkylpolyglucoside tel que défini ci-dessus avec l'alcool gras correspondant peut être sous forme d'une composition autoémulsionnante, comme décrit par exemple dans le document WO-A-92/06778.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

Les compositions selon l'invention trouvent leur application dans un grand nombre de traitements, notamment cosmétiques, de la peau, des lèvres et des cheveux, y compris le cuir chevelu, notamment pour la protection et/ou le soin de la peau, des lèvres et/ou des cheveux, et/ou pour le maquillage de la peau et/ou des lèvres.

Un autre objet de la présente invention est constitué par l'utilisation des compositions selon l'invention telles que ci-dessus définies pour la fabrication de produits pour le traitement cosmétique de la peau, des lèvres, des ongles, des cheveux, des cils, sourcils et/ou du cuir chevelu, notamment des produits de soin et des produits de maquillage.

Les compositions cosmétiques selon l'invention peuvent par exemple être utilisées comme produit de soin et/ou de protection solaire pour le visage et/ou le corps de consistance liquide à semi-liquide, telles que des laits, des crèmes plus ou moins onctueuses, gel-crèmes, des pâtes. Elles peuvent éventuellement être conditionnées en aérosol et se présenter sous forme de mousse ou de spray.

Les compositions selon l'invention sous forme de lotions fluides vaporisables conformes à l'invention sont appliquées sur la peau ou les cheveux sous forme de fines particules au moyen de dispositifs de pressurisation. Les dispositifs conformes à l'invention sont bien connus de l'homme de l'art et comprennent les pompes non-aérosols ou "atomiseurs", les récipients aérosols comprenant un propulseur ainsi que les pompes aérosols utilisant l'air comprimé comme propulseur. Ces derniers sont décrits dans les brevets US 4,077,441 et US 4,850,517 (faisant partie intégrante du contenu de la description).

Les compositions conditionnées en aérosol conformes à l'invention contiennent en général des agents propulseurs conventionnels tels que par exemple les composés hydrofluorés le dichlorodifluorométhane, le difluoroéthane, le diméthyléther, l'isobutane, le n-butane, le propane, le trichlorofluorométhane. Ils sont présents de préférence dans des quantités allant de 15 à 50% en poids par rapport au poids total de la composition.

Un autre objet de l'invention est l'utilisation d'un composé de formule (I) tel que définie ci-dessus dans une composition cosmétique ou dermatologique comme agent filtrant les radiations UV.

Un autre objet de l'invention est l'utilisation d'un composé de formule (I) tel que définie ci-dessus dans une composition cosmétique comme agent de contrôle de ta variation de la couleur de la peau due aux rayonnements UV.

Des exemples concrets, mais nullement limitatifs, illustrant l'invention, vont maintenant être donnés.

### EXEMPLE 1 : Préparation du 2.4-bis-(4'-amino benzalmalonate de di-néopentyle)-6-(4 "-amino benzoate de butyle-s-triazine de formule (1):

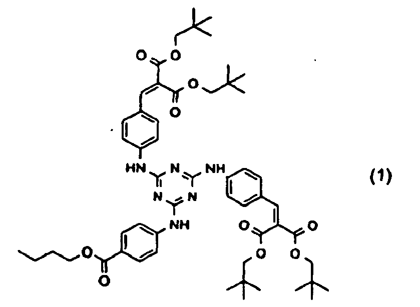

### Première étape : préparation du 2,4-dichloro-6-(4'-amino benzoate de butyle)-s-triazine

Dans un réacteur, on solubilise à 0-5°C le chlorure de cyanuryle (20,7 g, 0,112 mole) dans 250 ml d'acétone. On y ajoute goutte à goutte à 0-5°C en 1 heure une solution de para aminobenzoate de butyle (21,7 g, 0,112 mole) dissout dans 70 ml d'acétone. Ensuite, on y ajoute du bicarbonate de sodium (9,4 g, 0,112 mole) dissout dans 70 ml d'eau. Le mélange hétérogène est laissé 2 heures à une température de 0-5°C. Le précipité formé est filtré, puis lavé à l'eau et à l'acétone. Après séchage sous vide, on obtient 37,2 g (Rendement 97%) du 2,4-dichloro-6-(4'-amino benzoate de butyle)-s-triazine sous forme d'une poudre blanche : UV (Ethanol/DMSO) : λₘₐₓ = 298 nm , E_{1%} = 940,
et utilisée telle quelle dans l'étape suivante.

### Deuxième étape : préparation du dérivé de l'exemple 1 :

Sous barbotage d'azote, un mélange du produit précédent (7,41 g, 0,0213 mole) et de para-amino benzalmalonate de dinéopentyle (14,66 g, 0,0422 mole) en suspension dans 60 ml de toluène est chauffé au reflux pendant 7 heures 30 minutes. On refroidit et ajoute du dichlorométhane. La phase organique est lavée avec une solution saturée de bicarbonate de sodium puis à l'eau. La phase organique est séchée puis concentrée sous pression réduite. L'huile orangée obtenue (17,8 g) est soumise à une séparation sur colonne de silice (éluant : Heptane/EtOAc 85 :15). On récupère des fractions propres sous forme de paillettes jaunes pâles du dérivé de l'exemple 1 (8,72 g, Rendement 43%) :
UV (Ethanol) :
λ = 370 nm , E_{1%} = 623; λₘₐₓ = 347 nm , E_{1%} = 847; λ = 300 nm , E_{1%} = 432.

### EXEMPLE 2 : Préparation du 2,4-bis-(4'-amino benzalmalonate de di-néopentyle)-6-(4 "-amino benzoate d'amyle)-s-triazine de formule (2) :

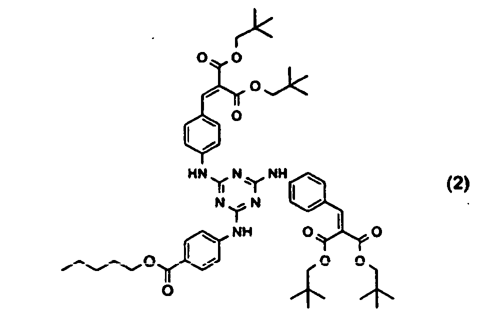

### Première étape : Préparation du 2,4-dichloro-6-(4'-amino benzoate d'amyle)-s-triazine :

Dans un réacteur on solubilise à 10°C le chlorure de cyanuryle (14,7 g, 0,0796 mole) dans 200 ml de dioxanne. On y ajoute simultanément goutte à goutte à 10°C en 1 heure une solution de para aminobenzoate d'amyle (16,5 g, 0,0796 mole) dissout dans 60 ml de dioxanne et une solution de carbonate de potassium (5,5 g, 0,0398 mole) dissout dans 30 ml d'eau. Le mélange hétérogène est laissé 2 heures à une température de 10°C. On ajoute environ 300 ml d'eau et le précipité formé est filtré, puis lavé à l'eau. Après séchage sous vide, on obtient 26,4 g (Rendement 93%) du 2,4-dichloro-6-(4'-amino benzoate d'amyle)-s-triazine sous forme d'une poudre blanche et utilisée telle quelle dans l'étape suivante.

### Deuxième étape : préparation du dérivé de l'exemple 2 :

Dans un micro ondes CEM Discover, on laisse le mélange intimement mélangé du produit précédent (0,103 g, 0,29x10⁻³ mole), de para-amino benzalmalonate de dinéopentyle (0,2 g, 0,58x10⁻³ mole) et de bicarbonate de sodium (0,049g, 0,58x10⁻³ mole) pendant 10 minutes à une température de 150°C et sous une puissance de 150 Watt. On extrait le solide amorphe formé au dichlorométhane. La phase organique est lavée 3 fois à l'eau et est séchée puis concentrée sous pression réduite. L'huile jaune obtenue est soumise à une séparation sur colonne de silice (éluant : Heptane/EtOAc 80 :20). On récupère des fractions propres sous forme d'une pâte jaune pâle du dérivé de l'exemple 2 (36 mg, Rendement 15%) :
UV (Ethanol) :
λ = 375 nm , E_{1%} = 610; λₘₐₓ = 347 nm , E_{1%} = 834; λ = 300 nm , E_{1%} = 425.

### EXEMPLE 3 : Préparation du 2,4-bis-(4'-amino benzalmalonate de di-néopentyle)-6-(4"-para-aminobenzoate d'éthyl-2-hexyle)-s-triazine de formule (3):

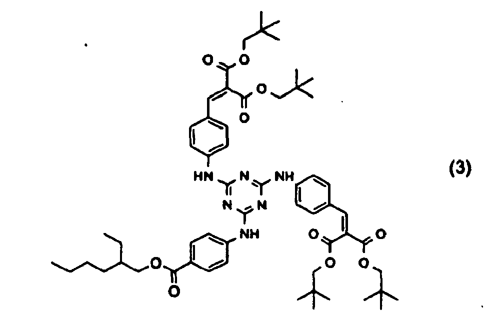

### Première étape: Préparation du 2,4-dichloro-6-(4'-amino benzoate d'éthyle-2-hexyle)-s-triazine :

Dans un réacteur, on solubilise à 0-5°C le chlorure de cyanuryle (18,4 g, 0,1 mole) dans 150 ml d'acétone. On y ajoute du bicarbonate de sodium (10,6 g, 0,1 mole) puis on ajoute goutte à goutte à une température inférieure à 10°C en 10 minutes une solution de para aminobenzoate d'éthyle-2-hexyle (24,9 g, 0,1 mole) dissout dans 150 ml d'acétone. Ensuite le mélange hétérogène est laissé 3 heures à la température du labo. On verse 500 ml d'eau. Le précipité formé est filtré, puis lavé à l'eau. Après séchage sous vide, on obtient 38 g d'un solide blanc cassé. Après recristallisation de ce solide dans le dichloro-1,2-éthane, on obtient 25,2g (Rendement 63%) du 2,4-dichloro-6-(4'-amino benzoate d'éthyle-2-hexyle)-s-triazine sous forme d'une poudre blanche : UV (Ethanol/DMSO) : λₘₐₓ = 291 nm , E_{1%} = 732,
et utilisée telle quelle dans l'étape suivante.

### Deuxième étape : préparation du dérivé de l'exemple 3 :

Sous barbotage d'azote, un mélange du produit précédent (1,14 g, 2,87x10⁻³ mole) et de para-amino benzalmalonate de dinéopentyle (2,2 g, 6,33x10⁻³ mole) en suspension dans 35 ml de toluène est chauffé au reflux pendant 10 heures 30 minutes. On refroidit et ajoute du dichlorométhane. La phase organique est lavée avec une solution saturée de bicarbonate de sodium puis à l'eau. La phase organique est séchée puis concentrée sous pression réduite. L'huile orangée obtenue (2,6 g) est soumise à une séparation sur colonne de silice (éluant : Heptane/EtOAc 85 :15). On récupère des fractions propres sous forme de paillettes jaunes pâles du dérivé de l'exemple 3 (1,17 g, Rendement 40%) :
UV (Ethanol) :
λ = 370 nm , E_{1%} = 575; λₘₐₓ = 342 nm , E_{1%} = 880; λ =300 nm , E_{1%} = 448.

### EXEMPLE 4 : Préparation du 2,4-bis-(4'-amino benzalmalonamide éthylester tert-butylamide)-6-(4"-para-aminobenzoate d'éthyl-2-hexyle)-s-triazine de formule (4):

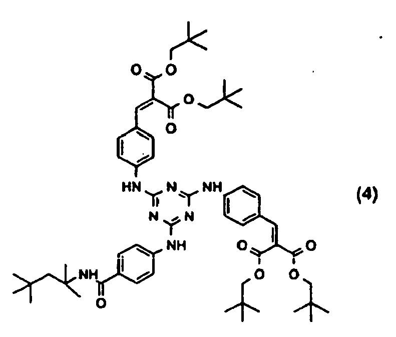

### Première étape : Préparation du 4-nitro-N-(tert-octyl) benzamide :

Dans un réacteur, on introduit de la tert-octyl amine (51,7 g, 0,4 mole) et de la triéthylamine (61,2 ml, 0,44 mole) dans 260 ml de dichloroéthane. On chauffe à 70°C puis on ajouteen 50 minutes le 4-nitrobenzoyl chloride (77,9 g, 0,42 mole) par petites portions. On chauffe au reflux pendant 4 heures.. On verse le mélange sur de l'eau glacée ; on extrait au dichlorométhane, sèche et évapore le solvant. Le précipité beige obtenu est recristallisé dans un mélange d'éther isopropylique et d'éthanol (rapport 10 :1). Après séchage sous vide, on obtient 84,6 g (Rendement 76%) du 4-nitro-N-(tert-octyl) benzamide sous forme d'une poudre blanc cassé et utilisée telle quelle dans l'étape suivante.

### Deuxième étape : préparation du 4-amino-N-(tert-octyl) benzamide :

Dans un hydrogénateur de 500 ml, du 4-nitro-N-(tert-octyl) benzamide (30 g, 0,108 mole) dissout dans 200 ml d'acétate d'éthyle est hydrogéné en présence de 4,8 g de palladium à 10% sur charbon à 50% d'eau comme catalyseur (pression d'hydrogène : 8-10 bars) à une température de 70-75°C pendant 1 heure et 15 minutes. Après filtration, concentration du solvant et séchage sous vide, on obtient 20,4 g (Rendement: 76%) de 4-amino-N-(tert-octyl) benzamide sous forme d'une poudre jaune clair et utilisée telle quelle dans l'étape suivante.

### Troisième étape: préparation du N-(tert-octyl)-4-[(4,6-dichloro-1,3,5-triazin-2yl)amino] benzamide :

Dans un réacteur, on solubilise à 10°C le chlorure de cyanuryle (3,7 g, 0,0201 mole) dans 70 ml de dioxanne. On y ajoute simultanément goutte à goutte à 10°C en 1 heure une solution du produit de l'étape précédente (5 g, 0,0201 mole) dissout dans 100 ml de dioxanne et une solution de carbonate de potassium (1,4 g, 0,03 mole) dissout dans 20 ml d'eau. Le mélange hétérogène est laissé 2 heures à une température de 10°C. On ajoute environ 300 ml d'eau et le précipité formé est filtré, puis lavé à l'eau. Après séchage sous vide, on obtient 7,4 g (Rendement 93%) du N-(tert-octyl)-4-[(4,6-dichloro-1,3,5-triazin-2-yl)amino] benzamide sous forme d'une poudre blanche et utilisée telle quelle dans l'étape suivante.

### Quatrième étape : préparation du dérivé de l'exemple 4 :

Dans un micro ondes CEM Discover, on laisse le mélange intimement mélangé du produit de l'étape précédente (0,29g 0,732x10⁻³ mole), de para-amino benzalmalonate de dinéopentyle (0,5g 1,44x10⁻³ mole) et de bicarbonate de sodium (0,14g 1,44x10⁻³ mole) pendant 4 minutes à une température de 60°C et sous une puissance de 300 Watt puis pendant 15 minutes à une température de 110°C. On extrait le solide amorphe formé au dichlorométhane. La phase organique est lavée 3 fois à l'eau et est séchée puis concentrée sous pression réduite. L'huile orangée obtenue est soumise à une séparation sur colonne de silice (éluant: Heptane/EtOAc 75:25). On récupère des fractions propres sous forme d'une huile jaune pâle qui se solidifie pour donner le dérivé de l'exemple 5 (0,6 g, Rendement 86%) sous forme d'une poudre jaune pâle:
UV (Ethanol) :
λₘₐₓ = 351 nm , E_{1%} = 763; λₘₐₓ = 298 nm , E_{1%} = 369.

### EXEMPLE 5: Préparation du 2.4-bis-(4'-amino benzalmalonate de di-néopentyle)-6-(4"-para-aminobenzamide de tert-butyle)-s-triazine de formule (5) :

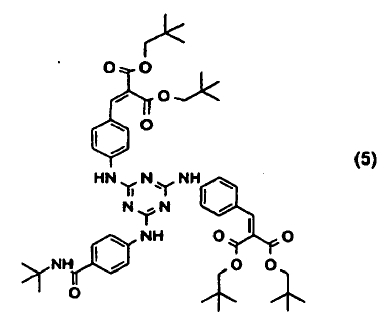

### Première étape : préparation du N-(tert-butyl)-4-nitro benzamide :

Dans un réacteur, on ajoute en 30 minutes à une température de 0-5°C du chlorure de 4-nitrobenzoyle (18,9 g, 0,1 mole) dissout dans 60 ml de chlorure de méthylène à une solution de tert-butylamine (8,3 g, 0,112 mole) et de triéthylamine (15,6 ml, 0,112 mole) dissouts dans 170 ml de dichlorométhane. Le mélange réactionnel est ramené à la température du labo et laissé sous agitation pendant 2 heures. La phase organique est lavée 2 fois à l'eau, séchée. Après élimination du solvant sous pression réduite, le solide obtenu est recristallisé dans l'isopropanol. On obtient 17,1 g (Rendement : 77%) de N-(tert-butyl)-4-nitro benzamide sous forme d'une poudre jaune pâle (Pf 161-2°C) et utilisé tel quel dans l'étape suivante.

### Deuxième étape : préparation de 4-amino-N-(tert-butyl) benzamide :

Dans un hydrogénateur de 1 litre, le produit précédent (17,1 g, 0,077 mole) dissout dans 300 ml d'isopropanol est hydrogéné en présence de 3 g de palladium sur charbon à 5% comme catalyseur (pression d'hydrogène : 7 bars) à une température de 60°C pendant 30 minutes. Après filtration, concentration du solvant et séchage sous vide, on obtient 13,2 g (Rendement : 89%) de 4-amino-N-(tert-butyl) benzamide sous forme d'une poudre gris clair (Pf 123-4°C) et utilisée telle quelle dans l'étape suivante.

### Troisième étape: préparation du N-(tert-butyl)-4-[(4,6-dichloro-1,3,5-triazin-2-yl)amino] benzamide :

Dans un réacteur, on solubilise à 10°C le chlorure de cyanuryle (11,71 g, 0,06 mole) dans 150 ml de dioxanne. On y ajoute simultanément goutte à goutte à 10°C en 1 heure une solution du produit de l'étape précédente (11,53 g, 0,06 mole) dissout dans 60 ml de dioxanne et une solution de carbonate de potassium (6,3 g, 0,03 mole) dissout dans 30 ml d'eau. Le mélange hétérogène est laissé 2 heures à une température de 10°C. On ajoute environ 300 ml d'eau et le précipité formé est filtré, puis lavé à l'eau. Après séchage sous vide, on obtient 18 g (Rendement 88%) du N-(tert-butyl)-4-[(4,6-dichloro-1,3,5-triazin-2-yl)amino] benzamide sous forme d'une poudre blanche (Pf 256-7°C) et utilisée telle quelle dans l'étape suivante.

### Quatrième étape: Préparation du dérivé de l'exemple 5 :

Dans un micro ondes CEM Discover, on laisse le mélange intimement mélangé du produit précédent (1,5 g, 4,4x10⁻³ mole), de para-amino benzalmalonate de dinéopentyle (3 g, 8,8x10⁻³ mole) et de bicarbonate de sodium (0,37 g, 8,8x10⁻³ mole) pendant 4 minutes à une température de 60°C et sous une puissance de 300 Watt puis pendant 20 minutes à une température de 150°C. On extrait le solide amorphe formé au dichlorométhane. La phase organique est lavée 3 fois à l'eau et est séchée puis concentrée sous pression réduite. L'huile marron obtenue est soumise à une séparation sur colonne de silice (éluant : Heptane/EtOAc 60 :40). On récupère des fractions propres sous forme d'une poudre jaune pâle du dérivé de l'exemple 5 (0,7 g, Rendement 17%) :
UV (Ethanol) :
λ = 375 nm , E_{1%} = 420; λₘₐₓ = 345 nm , E_{1%} = 813; λ = 299 nm , E_{1%} = 420.

### EXEMPLE 6 : Préparation du 2,4-bis-(4'-amino benzalmalonate de 1,3-diméthylebutyle)-6-(4"-para-aminobenzoate d'amyle)-s-triazine de formule (7) :

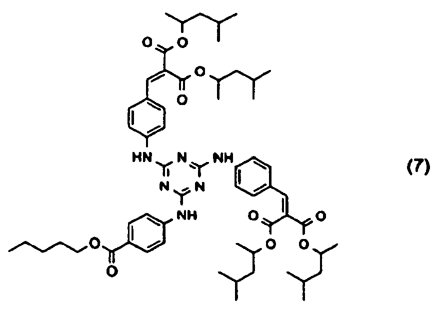

### Première étape : Préparation du malonate de 1,3-diméthylbutyle :

Dans un réacteur surmonté par un Dean Stark, on porte au reflux pendant 2 heures l'acide malonique (72,8 g, 0,7 mole) et l'alcool 2-méthyl-4-pentanol (286 g, 2,8 mole) dans 200 ml de toluène en présence de 1,8 ml d'acide sulfurique concentré. L'eau formée est éliminée par azéotropie. La phase organique est lavée 3 fois à l'eau et est séchée sur sulfate de sodium. On filtre et on évapore le solvant. Le produit obtenu distille à 147°C sous 20 hPa. On obtient 160 g (Rendement 79 %) du màlonate de 1,3-diméthylbutyle sous forme d'une huile incolore qui est utilisée telle quelle dans l'étape suivante.

### Deuxième étape : préparation du 4-nitrobenzalmalonate de 1,3-diméthylbutyle :

Dans un ballon équipé d'un Dean Stark surmonté d'un réfrigérant et sous barbotage d'azote, on place le p-nitro benzaldéhyde (49,9 g, 0,33 mole) et le malonate de 1,3-diméthylbutyle (90 g, 0,33 mole) dans 150 ml de toluène. On y ajoute le catalyseur préparé par avance, l'acide acétique (1,9 ml) et la pipéridine (3,3 ml) en suspension dans 4 ml de toluène. On porte le mélange sous agitation au reflux pendant 7 heures 30 minutes et élimine par l'intermédiaire du Dean Stark l'eau formée. Deux rajouts des mêmes quantités de catalyseur ont été nécessaires. On verse le mélange réactionnel refroidi dans l'eau et extrait au dichlorométhane. La phase organique est lavée à l'eau, puis séchée et concentrée sous pression réduite. L'huile marron rouge obtenue est chromatographiée sur colonne de silice (éluant : Heptane/EtOAc 97:3). On récupère 56,8 g (Rendement 43%) des fractions propres de 4-nitrobenzalmalonate de 1,3-diméthylbutyle sous forme d'une huile jaune et utilisée telle quelle dans l'étape suivante.

### Troisième étape : préparation du 4-aminobenzalmalonate de 1,3-diméthylbutyle

Sous agitation et barbottage d'azote, on disperse le dérivé de l'étape précédente (56,8 g, 0,14 mole) dans 80 ml d'acide acétique. On y ajoute 115 ml d'eau. Le mélange est chauffé à 50°C. On y ajoute le fer (78,2 g, 1,4 mole) par portions sans dépasser une température de 55°C (temps d'introduction 1 heure). Ensuite, on ajoute goutte à goutte de l'acide acétique (115 ml) sans dépasser une température de 55°C (temps d'introduction 2 heures). On chauffe 1 heure de plus à 55°C. On refroidit, ajoute de l'eau et extrait 2 fois au dichlorométhane. La phase organique est lavée à l'eau, avec une solution saturée de bicarbonate de sodium, à l'eau puis est séchée sur sulfate de sodium. Après concentration sous pression réduite, on obtient une huile marron rouge que l'on purifie par passage sur colonne de silice (éluant : Heptane/EtOAc 85 :15). On le recristallise dans un mélange d' heptane et de dichloro-1,2 éthane. On récupère 22,5 g (Rendement 43%) des fractions propres de 4-aminobenzalmalonate de 1,3-diméthylbutyle sous forme d'une huile orangé et utilisée telle quelle dans l'étape suivante.

### Quatrième étape: préparation du dérivé de l'exemple 6 :

Dans un micro ondes CEM Discover, on laisse le mélange intimement mélangé du produit de l'étape précédente (0,5 g. 1,32x10⁻³ mole), du produit de la deuxième étape de l'exemple 2 (0,235 g, 0.66x10⁻³ mole) et de bicarbonate de sodium (0.11 g, 1.32x10⁻³ mole) pendant 4 minutes à une température de 60°C et sous une puissance de 300 Watt puis pendant 25 minutes à une température de 110°C On extrait le solide amorphe formé au dichlorométhane. La phase organique est lavée 3 fois à l'eau et est séchée puis concentrée sous pression réduite. L'huile orangée obtenue est soumise à une séparation sur colonne de silice (éluant : Heptane/EtOAc 80 :20). On récupère des fractions propres sous forme d'une huile jaune qui se solidifie pour donner le dérivé de l'exemple 6 (0,4 g. Rendement 57%) sous forme de paillettes jaune clair:
UV (Ethanol) :
λ = 370 nm , E_{1%} = 500; λₘₐₓ = 337 nm , E_{1%}=800; λ= 300 nm, E_{1%}=411.

### EXEMPLE 7 : Préparation du 2.4-bis-(4'-amino benzalmalonate de di-menthyle)-6-(4"-para-aminobenzoate d'amyle)-s-triazine de formule (8):

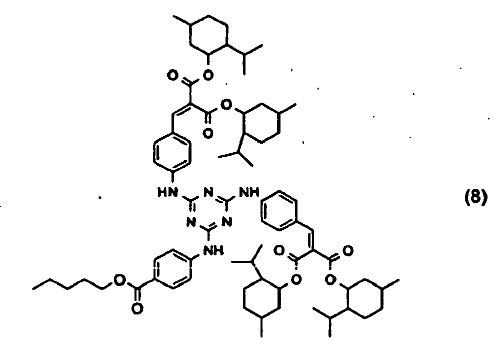

### Première étape: préparation du malonate de di-menthyle :

Dans un réacteur surmonté par un Dean Stark, on porte au reflux pendant 6 heures l'acide malonique (22,2 g, 0,213 mole) et le menthol (70 g, 0,448 mole) dans 100 ml de toluène en présence de 2 ml d'acide sulfurique concentré. L'eau formée est éliminée par azéotropie. La phase organique est lavée 3 fois à l'eau et est séchée sur sulfate de sodium. On élimine l'excès de menthol par distillation sous vide (140°C sous 0,6 hPa). Le résidu est traité au noir animal dans l'isopropanol au reflux. Après filtration, riçage et évaporation du solvant, on obtient 61,8 g (Rendement 76 %) du malonate de di menthyle sous forme d'une huile jaune qui est utilisée telle quelle dans l'étape suivante.

### Deuxième étape : préparation du 4-nitrobenzalmalonate de 1,3-diméthylbutyle :

Dans un ballon équipé d'un Dean Stark surmonté d'un réfrigérant et sous barbotage d'azote, on place le p-nitro benzaldéhyde (21,8 g, 0,144 mole) et le malonate de di menthyle (61 g, 0,16 mole) dans 100 ml de toluène. On y ajoute le catalyseur préparé par avance, l'acide acétique (0,92 ml) et la pipéridine (1,6 ml) en suspension dans 3 ml de toluène. On porte le mélange sous agitation au reflux pendant 9 heures et élimine par l'intermédiaire du Dean Stark l'eau formée. Trois rajouts des mêmes quantités de catalyseur ont été nécessaires. On verse le mélange réactionnel refroidi dans l'eau et extrait au dichlorométhane. La phase organique est lavée à l'eau, puis séchée et concentrée sous pression réduite. L'huile marron rouge obtenue est chromatographiée sur colonne de silice (éluant : Heptane/EtOAc 95:5). On récupère 37 g (Rendement 50%) des fractions propres de 4-nitrobenzalmalonate de di menthyle sous forme d'une huile jaune et utilisée telle quelle dans l'étape suivante.

### Troisième étape : préparation du 4-aminobenzalmalonate de 1,3-diméthylbutyle

Sous agitation et barbotage d'argon, on disperse le dérivé de l'étape précédente (37 g, 0,072 mole) dans 30 ml d'acide acétique et 45 ml d'eau. Le mélange est chauffé à 50°C. On y ajoute le fer (24,4 g, 0,437 mole) par portions sans dépasser une température de 55°C (temps d'introduction 30 minutes). Ensuite, on ajoute goutte à goutte de l'acide acétique (45 ml) sans dépasser une température de 55°C (temps d'introduction 1 heure 30 minutes). On chauffe 1 heure de plus à 55°C. On refroidit, ajoute de l'eau et extrait 2 fois au dichlorométhane. La phase organique est lavée à l'eau, avec une solution saturée de bicarbonate de sodium, à l'eau puis est séchée sur sulfate de sodium. Après concentration sous pression réduite, on obtient une gomme orangée que l'on purifie par passage sur colonne de silice (éluant : Heptane/EtOAc 90 :10). On récupère 8,6 g (Rendement 25%) des fractions propres de 4-aminobenzalmalonate de di menthyle sous forme d'un solide jaune et utilisé telle quelle dans l'étape suivante.

### Quatrième étape : préparation du dérivé de l'exemple 7 :

Dans un micro ondes CEM Discover, on laisse le mélange intimement mélangé du produit précédent (2 g, 4,1x10⁻³ mole), de 2,4-dichloro-6-(4'-amino benzoate d'amyle)-s-triazine (première étape de l'exemple 2) (0,73 g, 2,05x10⁻³ mole) et de bicarbonate de sodium (0,34 g, 4,1x10⁻³ mole) pendant 30 minutes à une température de 130-140°C et sous une puissance de 300 Watt. On extrait le solide amorphe formé au dichlorométhane. La phase organique est lavée 3 fois à l'eau et est séchée puis concentrée sous pression réduite. L'huile marron obtenue est soumise à une séparation sur colonne de silice (éluant : Heptane/EtOAc 80 :20). On récupère des fractions propres sous forme d'une poudre jaune pâle du dérivé de l'exemple 7 (0,5 g, Rendement 16%) :
UV (Ethanol) :
λ = 370 nm, E_{1%}=391; λₘₐₓ=340 nm, E_{1%}=538; λ=300 nm, E_{1%}=313.

### EXEMPLE A : EMULSION H/E

| **PHASES** | **INGREDIENTS** | **QUANTITES (G%)** |
|---|---|---|
| PHASE 1 | MELANGE MONO-STEARATE DE GLYCERYLE / STEARATE DE POLYETHYLENE GLYCOL (100 OE) | 1 |
| | ACIDE STEARIQUE | 1.5 |
| | POLY DIMETHYLSILOXANE | 0.5 |
| | ALCOOL CETYLIQUE | 0.5 |
| | MELANGE CETYLSTEARYL GLUCOSIDE / ALCOOL CETYLSTEARYLIQUE | 2 |
| | CONSERVATEUR | 1 |
| | TRIETHANOLAMINE | 0.45 |
| | BENZOATE D'ALCOOLS C₁₂/C₁₅ | 15 |
| | COMPOSE DE FORMULE (1) | 1 |
| PHASE 2 | ACIDE ETHYLENE DIAMINE TETRACETIQUE, SEL DISODIQUE, 2 H₂O | 0.1 |
| | PHOSPHATE DE MONO-CETYLE MONO-POTASSIQUE | 1 |
| | GLYCERINE | 5 |
| | GOMME DE XANTHANE | 0.2 |
| | EAU DESIONISEE (QS) | 68.35 |
| PHASE 3 | ACRYLATES/C10-30 ALKYL ACRYLATECROSSPOLYMER | 0,2 |
| | ISO-HEXADECANE | 1 |
| PHASE 4 | TRIETHANOLAMINE | 0,2 |
| | EAU DESIONISEE | 1 |

### EXEMPLE B : EMULSION H/E

| **PHASES** | **INGREDIENTS** | **QUANTITES (G%)** |
|---|---|---|
| PHASE 1 | MELANGE MONO-STEARATE DE GLYCERYLE / STEARATE DE POLYETHYLENE GLYCOL (100 OE) | 1 |
| | ACIDE STEARIQUE | 1.5 |
| | POLY DIMETHYLSILOXANE | 0.5 |
| | ALCOOL CETYLIQUE | 0.5 |
| | MELANGE CETYLSTEARYL GLUCOSIDE / ALCOOL CETYLSTEARYLIQUE | 2 |
| | CONSERVATEUR | 1 |
| | TRIETHANOLAMINE | 0.45 |
| | BENZOATE D'ALCOOLS C₁₂/C₁₅ | 15 |
| | COMPOSE DE FORMULE (1) | 5 |
| PHASE 2 | ACIDE ETHYLENE DIAMINE TETRACETIQUE, SEL DISODIQUE, 2 H₂O | 0.1 |
| | PHOSPHATE DE MONO-CETYLE MONO-POTASSIQUE | 1 |
| | GLYCERINE | 5 |
| | GOMME DE XANTHANE | 0.2 |
| | EAU DESIONISEE (QS) | 64.35 |
| PHASE 3 | ACRYLATES/C10-30 ALKYL ACRYLATECROSSPOLYMER | 0.2 |
| | ISO-HEXADECANE | 1 |
| PHASE 4 | TRIETHANOLAMINE | 0.2 |
| | EAU DESIONISEE | 1 |

### PHOTOSTABILITES COMPAREES ENTRE UN COMPOSE DE L'ART ANTERIEUR ET LE COMPOSE SELON L'INVENTION DE L'EXEMPLE 1.

### Produits testés :

2,4,6-tris(4'-amino benzalmalonate de diisobutyle)-s-triazine (exemple 1 du brevet EP 0507691).

2,4-bis-(4'-amino benzalmalonate de di-néopentyle)-6-(4'-amino benzoate de butyle)-s-triazine = composé de formule (1) selon l'invention.

Les deux produits ont été mis en solution à 2% en poids dans l'huile Miglyol 812. Environ 10 mg de solution huileuse sont étalés sur 10 cm² à la surface d'un disque creux de verre dépoli ; la quantité est déterminée par pesée.

Les films des solutions huileuses sont irradiés une heure à l'aide d'un simulateur solaire ORIEL (UVA = 14,2 mW/cm² ; UVB = 0,41 mW/cm²), puis extraits par 10 ml d'éthanol à10% d'isopropanol et passage 5 min aux ultrasons. Les quantifications des produits sont faites par HPLC des extraits.

Conditions HPLC : colonne : UP5WOD-25QS, 250*4.6 mm, 5 µm, Interchrom ; éluant : méthanol (exemple comparatif 1) et 98% de méthanol + 2% d'eau (exemple 1) ; Débit : 1 ml/mn ; volume injecté : 10 µl ; détection : Barrette de diodes ; tr (min) : 5.2 (composé de l'art antérieur) et 7,4 (composé de l'exemple 1).

Les taux de perte sont déterminés par comparaison des quantités de produit présent dans les échantillons irradiés et dans les témoins non irradiés préparés simultanément et traités de la même façon (moyennes sur 3 échantillons ; S = surface / mg solution) : % perte = 100 *(S0―Sirr) / S0

### RESULTATS DE PHOTOSTABILITE

| **Composés** | **% de disparition (perte moyenne)** |
|---|---|
| Composé (art antérieur) | 10.0 |
| Composé de formule (1) (invention) | 4.6 |

## Revendications

1. Composé s-triazine répondant à la formule générale (I) suivante : 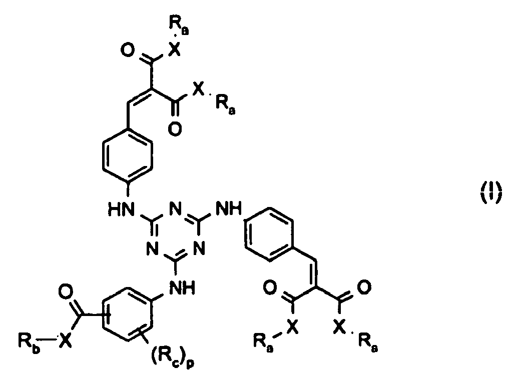 dans laquelle :
X, identiques ou différents, désignent -0- ou -NR₆-
Rₐ, identiques ou différents, désignent un groupe de formule (II) : 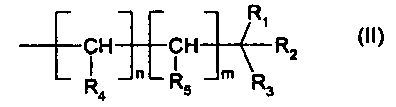 dans laquelle :
R₁ et R₂, identiques ou différents représentent un groupe alkyle en C₁-C₈, linéaire ou ramifié,
R₁ et R₂ peuvent former un cycle en C₅-C₈, éventuellement substitué par 1, 2 ou 3 groupements alkyle(s) en C₁-C₄, linéaire(s) ou ramifié(s) ;
R₃, R₄ et R₅, identiques ou différents représentent un atome d'hydrogène ou un groupe alkyle en C₁-C₄, linéaire ou ramifié :
n vaut 0 ou 1 ;
m vaut 0 ou 1 ;
sous réserve que :
(i) lorsque n = 1 et R₄ désigne l'hydrogène alors m est égal à 0 et ,
(ii) lorsque R₁ et R₂ forment un cycle en C₅-C₈ alors la somme n+m est différente de 2 ;
R₆ représente l'hydrogène ou un groupe alkyle en C₁-C₈,
R_{b} désigne un groupe alkyle en C₁-C₂₀, linéaire ou ramifié et éventuellement insaturé,
un groupe cycloalkyle en C₅-C₁₂, éventuellement substitué par 1 à 3 radicaux alkyles en C₁-C₄, linéaires ou ramifiés, le groupe -(CH₂CHR₇-O)_{q}R₈ ou le groupe -CH₂-CH(OH)-CH₂-O-R₈,
R₇ représente l'hydrogène ou méthyle,
R₈ représente l'hydrogène ou un groupe alkyle en C₁-C₈,
q = 1-20,
le groupement COXR_{b} peut être en position ortho, méta ou para du groupement amino,
R_{c} désigne un radical alkyle en C₁-C₂₀, linéaire ou ramifié, saturé ou insaturé, le radical OH, un radical alcoxy en C₁-C₂₀, linéaire ou ramifié,
p est égal à 0, 1 ou 2, **caractérisé par le fait qu'**il est choisi parmi
(1) ceux pour lesquels les deux conditions suivantes sont réunies :
(a) n=m=0 et
(b) R₁, R₂, R₃ désignent un alkyle en C₁-C₄.
ou bien R₃ désigne hydrogène et R₁ et R₂ forment un cycle en C5-C8 éventuellement substitué par 1 ou deux radicaux alkyle ;
(2) ceux pour lesquels les deux conditions suivantes sont réunies :
(a) n=1 et R₄ désigne un alkyle en particulier méthyle ou m = 1 et R₅ désigne un alkyle en particulier méthyle et
(b) R₁ et R₂ désignent un alkyle en C₁-C₄ et plus particulièrement méthyle ;
(3) les composés choisis parmi : 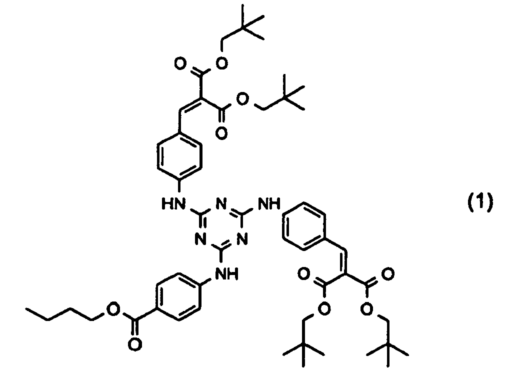 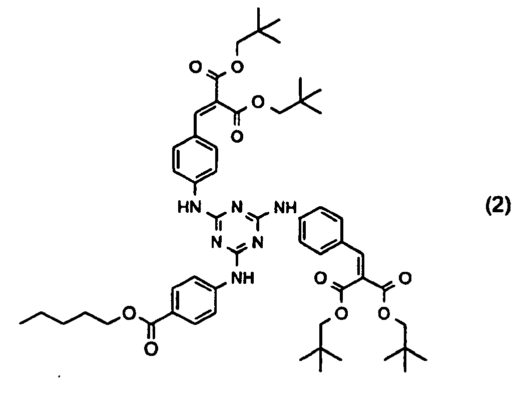 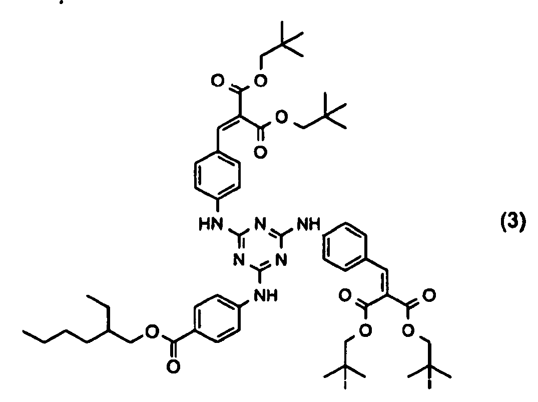 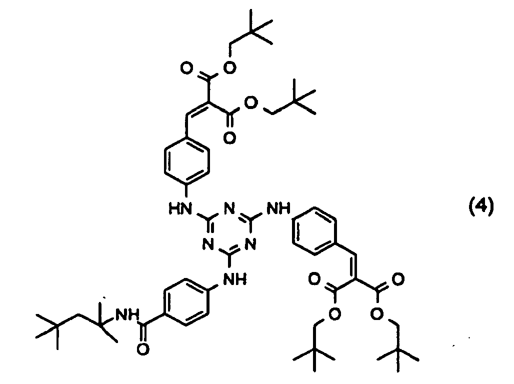 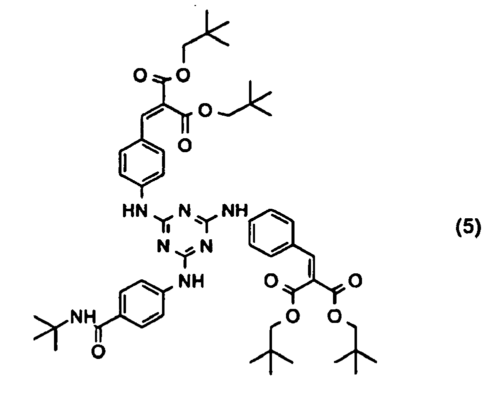 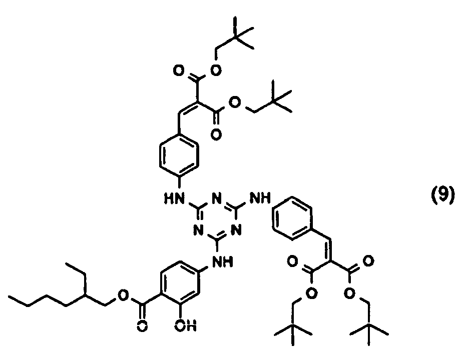 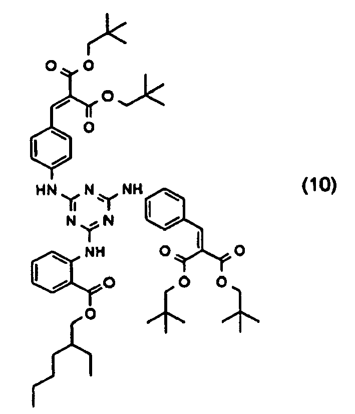
(4) le composé (6) de formule:

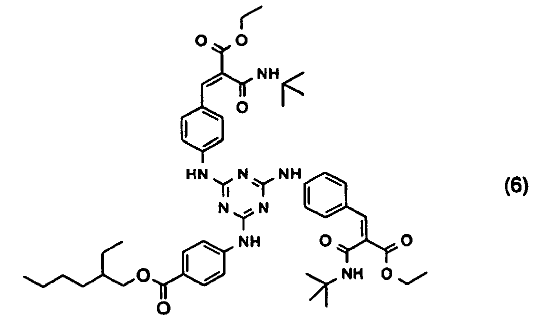

2. Composé selon la revendication 1, **caractérisé par le fait qu'**il est choisi parmi pour lesquels les deux conditions suivantes sont réunies :
(a) n=m=0 et
b) R₁, R₂, R₃ désignent méthyle
ou bien R₃ désigne hydrogène et R₁ et R₂ forment un cycle cyclohexyle.

3. Composé selon la revendication 1 4, **caractérisé par le fait qu'**il est choisi parmi pour lesquels les deux conditions suivantes sont réunies :
(a) n=1 et R₄ désigne méthyle ou m = 1 et R₅ désigne méthyle et
(b) R₁ et R₂ désignent méthyle.

4. Composé selon l'une quelconque des revendication1 1 à 3, **caractérisé par le fait qu'**il est choisi parmi les composés de formules (1) à (10) suivantes :

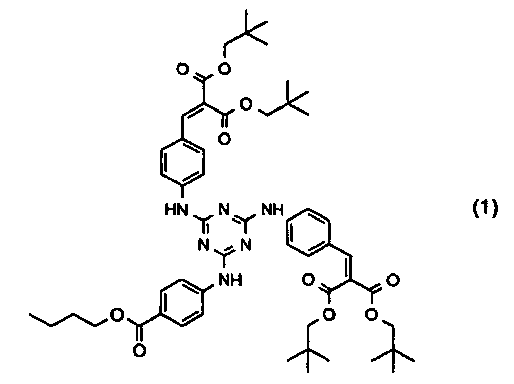

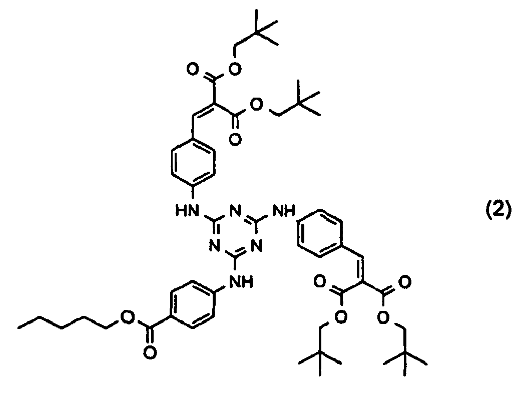

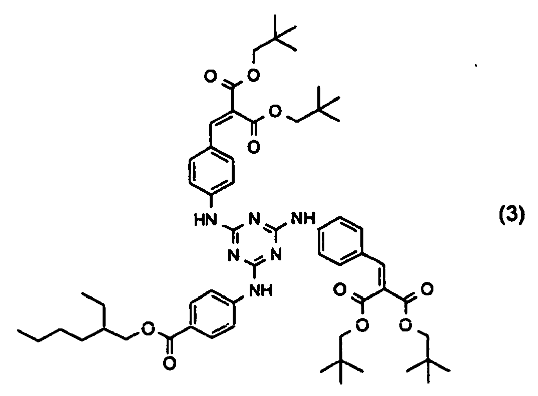

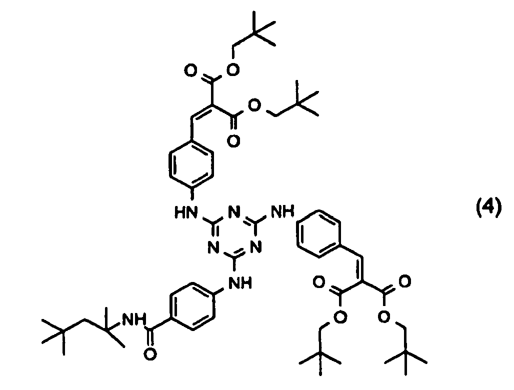

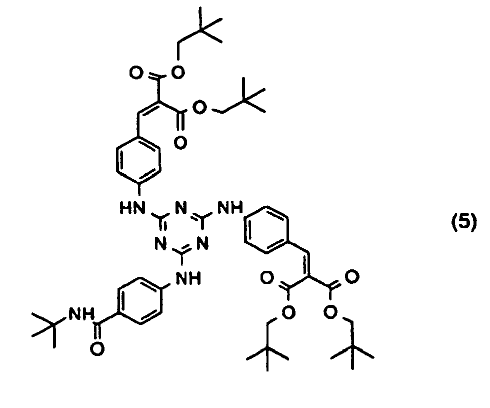

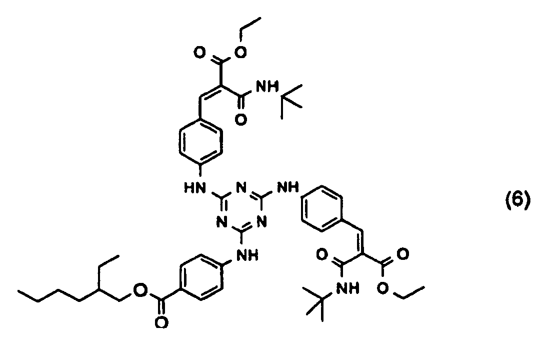

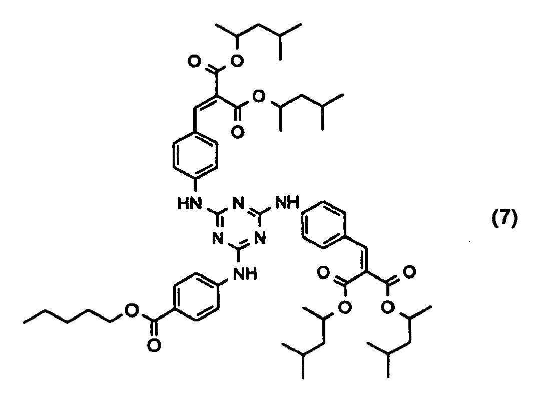

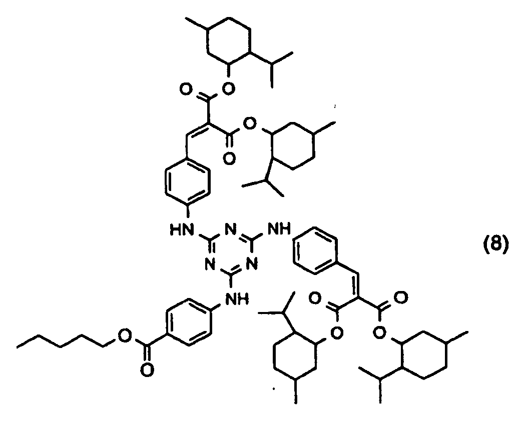

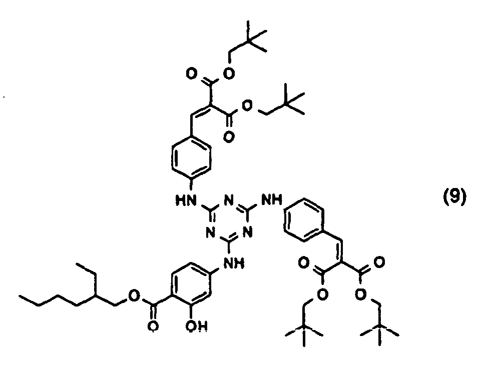

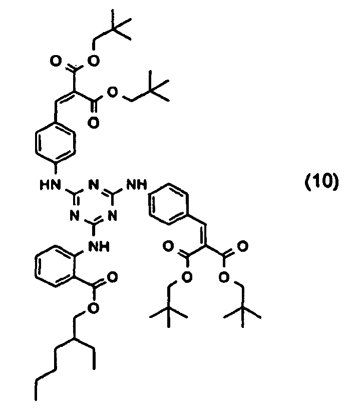

5. Composé selon la revendication 4, **caractérisé par le fait qu'**il s'agit du 2,4-bis-(4'-amino benzalmalonate de di-néopentyle)-6-(4"-amino benzoate de butyle)-s-triazine de formule (1).

6. Composition cosmétique ou dermatologique, contenant dans un milieu physiologiquement acceptable au moins un composé de formule (I) tel que défini dans l'une des revendications précédentes.

7. Composition selon la revendication 6, où le ou les composés de formule (I) sont présents dans des proportions allant de 0,01 % à 20 % en poids, de préférence de 0,1 % à 10 % en poids, par rapport au poids total de la composition.

8. Composition selon la revendication 6 ou 7, **caractérisée par le fait qu'**elle contient en plus d'autres agents photoprotecteurs actifs dans l'UV-A et/ou l'UV-B.

9. Composition selon l'une quelconque des revendications 6 à 8, **caractérisée par le fait qu'**elle comprend en outre au moins un agent de bronzage et/ou de brunissage artificiel de la peau.

10. Composition selon l'une quelconque des revendications 6 à 9, **caractérisée par le fait qu'**elle comprend en outre au moins un adjuvant choisi parmi les corps gras, les solvants organiques, les épaississants ioniques ou non ioniques, hydrophiles ou lipophiles, les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, des actifs, les charges, les polymères, les propulseurs, les agents alcalinisants ou acidifiants

11. Utilisation d'une composition telles que définie dans l'une quelconque des revendications 6 à 10 pour la fabrication de produits pour le traitement cosmétique de la peau, des lèvres, des ongles, des cheveux, des cils, sourcils et/ou du cuir chevelu

12. Utilisation d'une composition telles que définie dans l'une quelconque des revendications 6 à 10 pour la fabrication de produits de soin de la peau, des lèvres, des ongles, des cheveux et/ou du cuir chevelu.

13. Utilisation d'une composition telles que définie dans l'une quelconque des revendications 7 à 10 pour la fabrication de produits de maquillage.

14. Utilisation d'un composé de formule (I) tel que défini dans les revendications 1 à 5 7 dans une composition cosmétique ou dermatologique comme agent filtrant les radiations UV.

15. Utilisation d'un composé de formule (I) tel que défini dans les revendications 1 à 5 7 comme agent de contrôle de la variation de la couleur de la peau due aux rayonnements UV.

## Claims

1. s-Triazine compound corresponding to the following general formula (I): 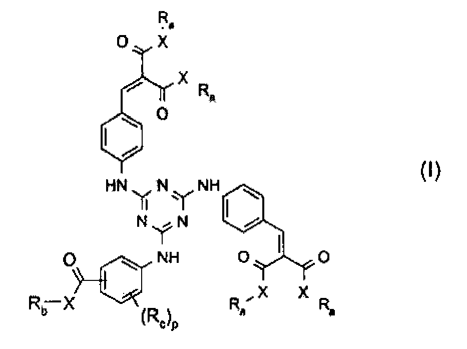 in which:
X, which are identical or different, denote -O- or -NR₆-,
Rₐ, which are identical or different, denote a group of formula (II): 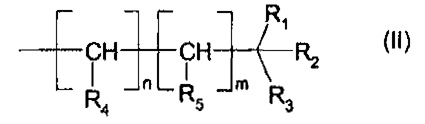 in which:
R₁ and R₂, which are identical or different, represent a linear or branched C₁-C₈ alkyl group,
R₁ and R₂ can form a C₅-C₈ ring, optionally substituted by 1, 2 or 3 linear or branched C₁-C₄ alkyl group(s);
R₃, R₄ and R₅, which are identical or different, represent a hydrogen atom or a linear or branched C₁-C₄ alkyl group;
n has the value 0 or 1;
m has the value 0 or 1;
with the proviso that:
(i) when n = 1 and R₄ denotes hydrogen, then m is equal to 0 and,
(ii) when R₁ and R₂ form a C₅-C₈ ring, then the sum n+m is other than 2;
R₆ represents hydrogen or a C₁-C₈ alkyl group,
R_{b} denotes a linear or branched and optionally unsaturated C₁-C₂₀ alkyl group, a C₅-C₁₂ cycloalkyl group optionally substituted by 1 to 3 linear or branched C₁-C₄ alkyl radicals, the -(CH₂CHR₇-O)_{q}R₈ group or the -CH₂-CH(OH)-CH₂-O-R₈ group,
R₇ represents hydrogen or methyl,
R₈ represents hydrogen or a C₁-C₈ alkyl group,
q = 1-20,
the COXR_{b} group can be in the ortho, meta or para position with respect to the amino group,
R_{c} denotes a saturated or unsaturated and linear or branched C₁-C₂₀ alkyl radical, the OH radical or a linear or branched C₁-C₂₀ alkoxy radical,
p is equal to 0, 1 or 2,
**characterized in that** it is chosen from
(1) those for which the following two conditions are combined:
(a) n = m = 0 and
(b) R₁, R₂ and R₃ denote a C₁-C₄ alkyl,
or else R₃ denotes hydrogen and R₁ and R₂ form a C₅-C₈ ring optionally substituted by one or two alkyl radicals.
(2) those for which the following two conditions are combined:
(a) n = 1 and R₄ denotes an alkyl, in particular methyl, or m = 1 and R₅ denotes an alkyl, in particular methyl, and
(b) R₁ and R₂ denote a C₁-C₄ alkyl and more particularly methyl;
(3) the compounds chosen from: 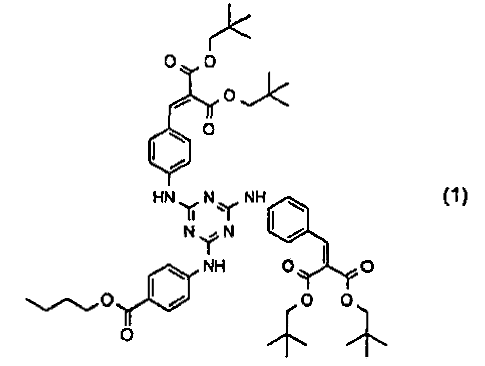 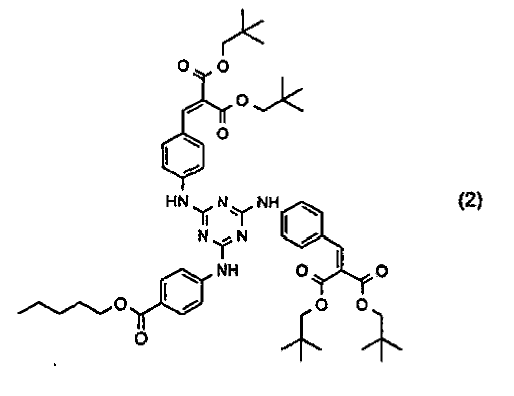 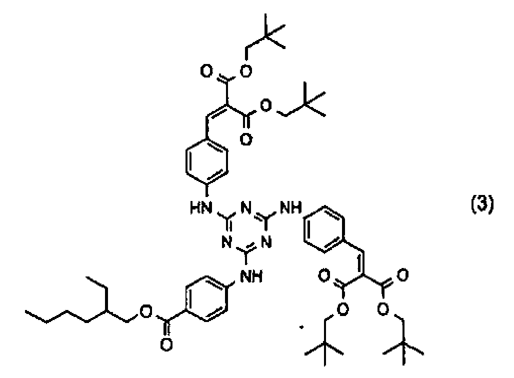 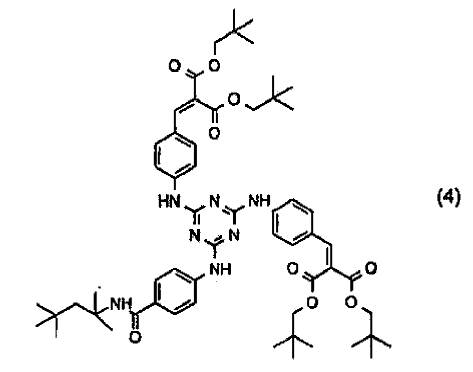 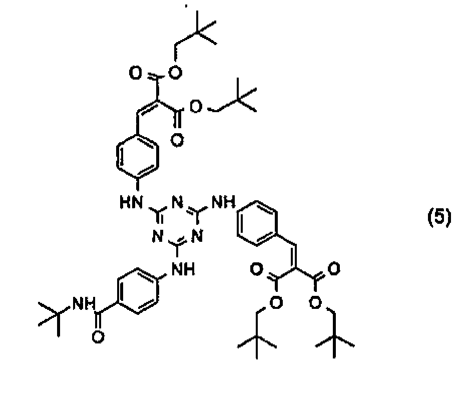 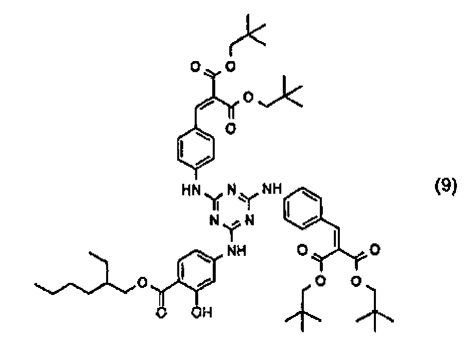 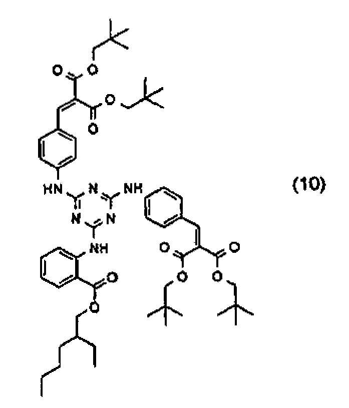
(4) the compound (6) of formula:

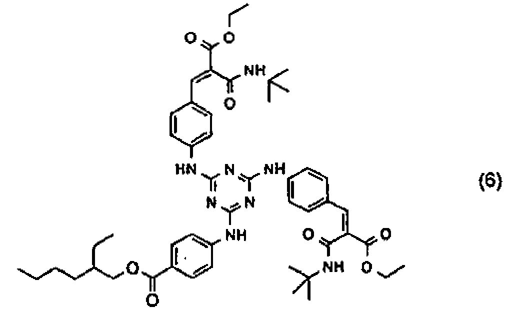

2. Compound according to Claim 1, **characterized in that** it is chosen from those for which the following two conditions are combined:
(a) n = m = 0 and
(b) R₁, R₂ and R₃ denote methyl,
or else R₃ denotes hydrogen and R₁ and R₂ form a cyclohexyl ring.

3. Compound according to Claim 1, **characterized in that** it is chosen from those for which the following two conditions are combined:
(a) n = 1 and R₄ denotes methyl or m = 1 and R₅ denotes methyl, and
(b) R₁ and R₂ denote methyl.

4. Compound according to any one of Claims 1 to 3, **characterized in that** it is chosen from the compounds of following formulae (1) to (10):

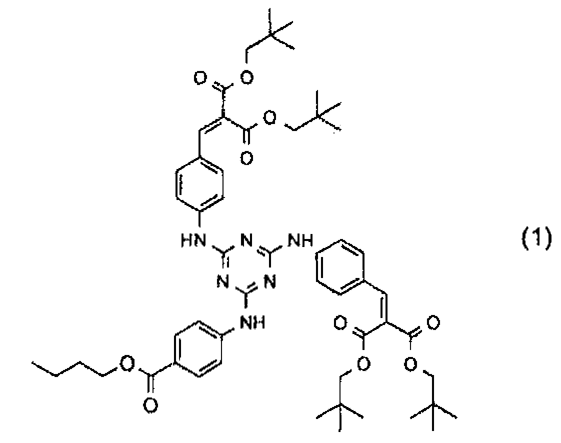

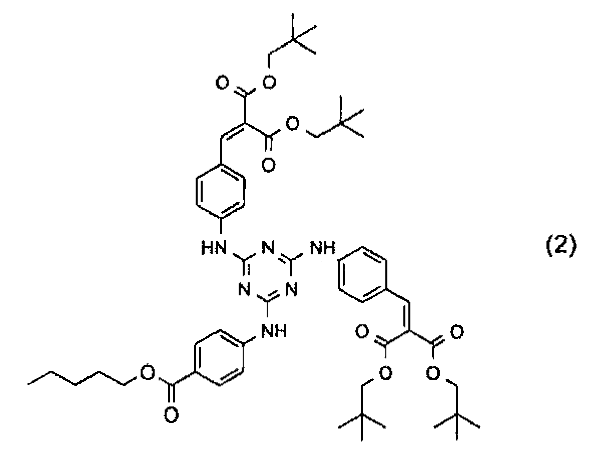

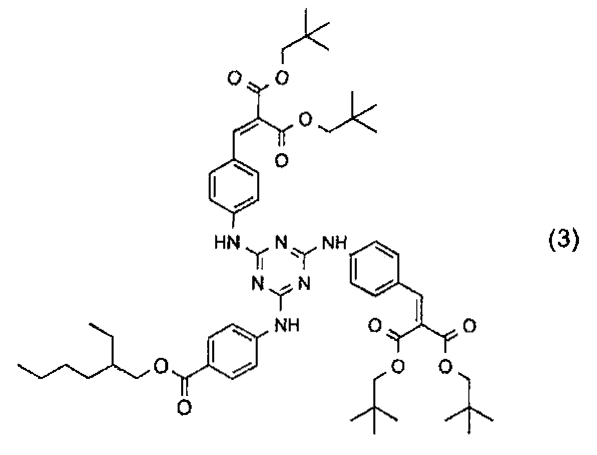

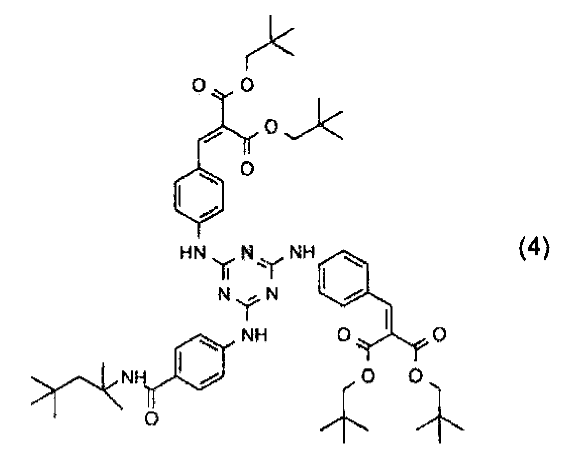

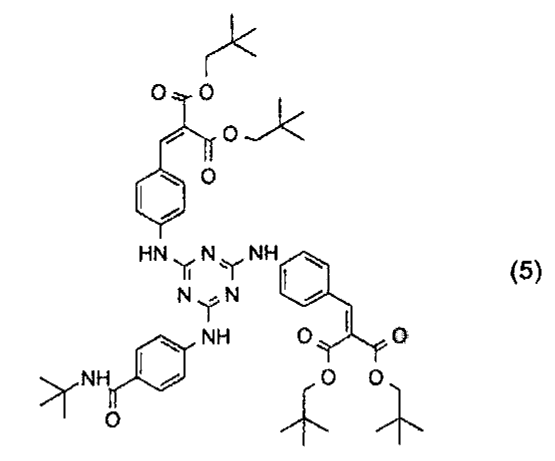

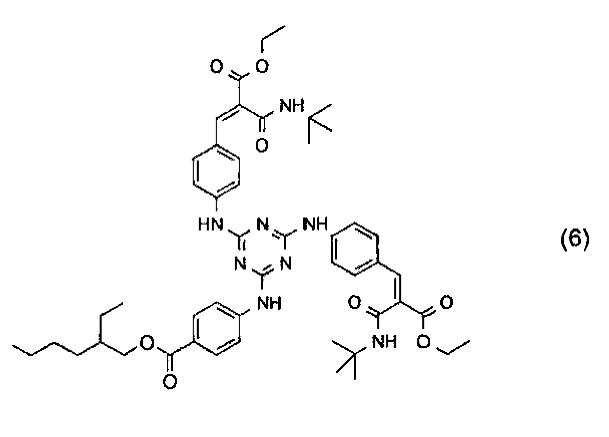

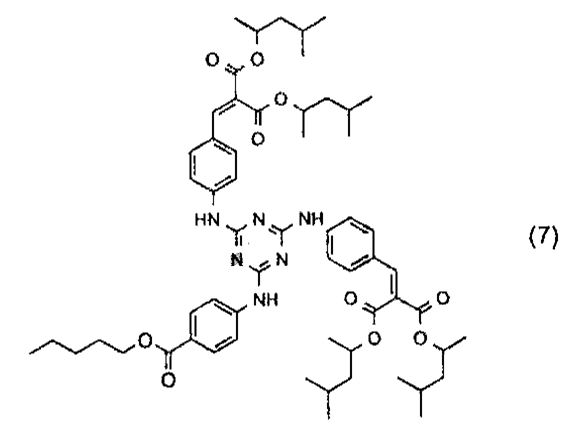

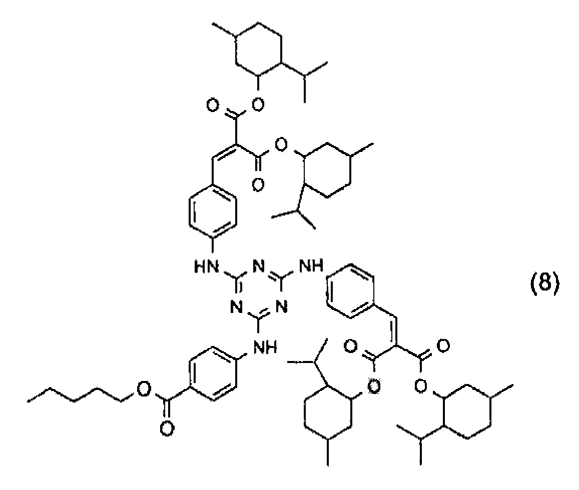

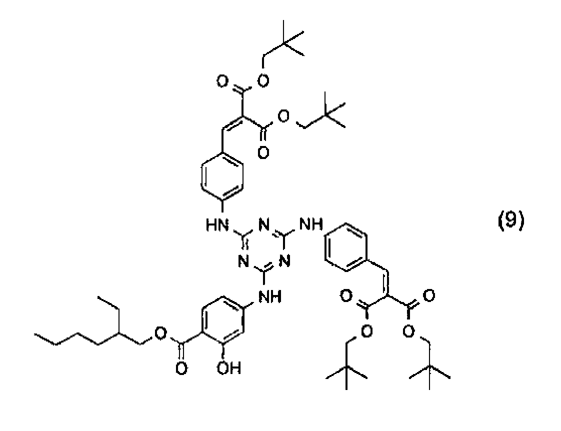

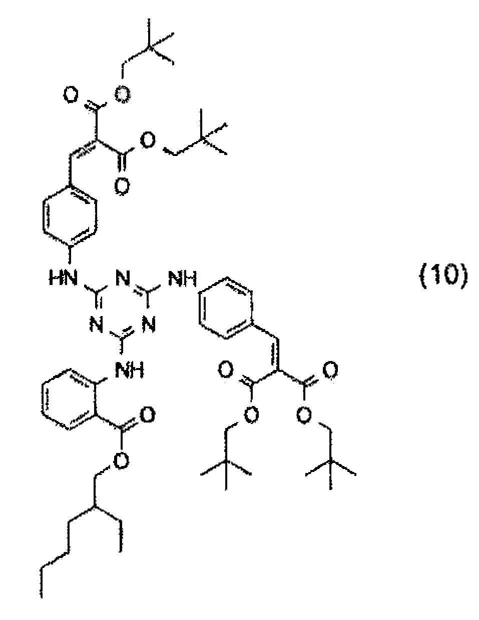

5. Compound according to Claim 4, **characterized in that** it is 2,4-bis(dineopentyl 4'-aminobenzalmalonate)-6-(butyl 4"-aminobenzoate)-s-triazine of formula (1).

6. Cosmetic or dermatological composition comprising, in a physiologically acceptable medium, at least one compound of formula (I) as defined in one of the preceding claims.

7. Composition according to Claim 6, where the compound or compounds of formula (I) are present in proportions ranging from 0.01% to 20% by weight, preferably from 0.1% to 10% by weight, with respect to the total weight of the composition.

8. Composition according to Claim 6 or 7, **characterized in that** it additionally comprises other photoprotective agents which are active in the UV-A and/or UV-B regions.

9. Composition according to any one of Claims 6 to 8, **characterized in that** it additionally comprises at least one agent for the artificial tanning and/or browning of the skin.

10. Composition according to any one of Claims 6 to 9, **characterized in that** it additionally comprises at least one adjuvant chosen from fatty substances, organic solvents, ionic or nonionic and hydrophilic or lipophilic thickeners, softeners, humectants, opacifiers, stabilizers, emollients, silicones, antifoaming agents, fragrances, preservatives, anionic, cationic, nonionic, zwitterionic or amphoteric surfactants, active principles, fillers, polymers, propellants or basifying or acidifying agents.

11. Use of a composition as defined in any one of Claims 6 to 10 in the manufacture of products for the cosmetic treatment of the skin, lips, nails, hair, eyelashes, eyebrows and/or scalp.

12. Use of a composition as defined in any one of Claims 6 to 10 in the manufacture of products for the care of the skin, lips, nails, hair and/or scalp.

13. Use of a composition as defined in any one of Claims 7 to 10 in the manufacture of makeup products.

14. Use of a compound of formula (I) as defined in Claims 1 to 5 in a cosmetic or dermatological composition as agent for screening out UV radiation.

15. Use of a compound of formula (I) as defined in Claims 1 to 5 as agent for controlling the variation in the colour of the skin due to UV radiation.

## Patentansprüche

1. s-Triazin der folgenden allgemeinen Formel (I) : 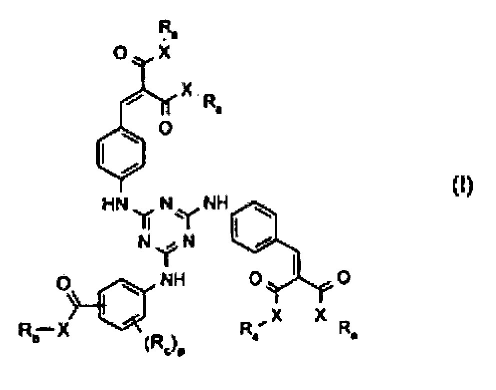 worin bedeuten:
die Gruppen X, die gleich oder verschieden sind, bedeuten -O- oder-NR₆-,
die Gruppen Rₐ, die gleich oder verschieden sind, bedeuten eine Gruppe der Formel (II): 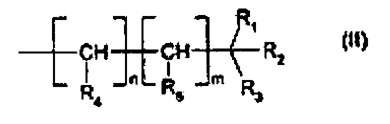 in der Formel:
R₁ und R₂, die gleich oder verschieden sind, bedeuten eine lineare oder verzweigte C₁-₈-Alkylgruppe,
R₁ und R₂ können einen C₅-₈-Ring bilden, der gegebenenfalls mit 1, 2 oder 3 linearen oder verzweigten C₁-₄-Alkylgruppe(n) substituiert ist,
R₃, R₄ und R₅, die gleich oder verschieden sind, bedeuten ein Wasserstoffatom oder eine lineare oder verzweigte C₁₋₄-Alkylgruppe;
n ist 0 oder 1;
m ist 0 oder 1;
mit der Maßgabe, dass
(i) wenn n = 1 und R₄ Wasserstoff bedeutet, ist m gleich 0 und
(ii) wenn R₁ und R₂ einen C₅₋₈-Ring bilden, ist die Summe n + m von 2 verschieden;
R₆ bedeutet Wasserstoff oder eine C₁₋₈-Alkylgruppe;
R_{b} bedeutet eine lineare oder verzweigte und gegebenenfalls ungesättigte C₁₋₂₀-Alkylgruppe, eine C₅₋₁₂-Cycloalkylgruppe, die gegebenenfalls mit 1 bis 3 linearen oder verzweigten C₁₋₄-Alkylgruppen substituiert ist, die Gruppe -(CH₂CHR₇-O)_{q}R₈ oder
die Gruppe -CH₂-CH(OH)-CH₂-O-R₈,
R₇ bedeutet Wasserstoff oder Methyl,
R₈ bedeutet Wasserstoff oder eine C₁₋₈-Alkylgruppe;
q = 1 - 20,
die Gruppe COXR_{b} kann sich in ortho-, meta- oder para-Stellung zur Aminogruppe befinden,
R_{c} bedeutet eine lineare oder verzweigte, gesättigte oder ungesättigte C₁₋₂₀-Alkylgruppe, die Gruppe OH, eine lineare oder verzweigte C₁₋₂₀-Alkoxygruppe,
p ist 0, 1 oder 2,
**dadurch gekennzeichnet, dass** die Verbindung ausgewählt ist unter:
(1) Verbindungen, bei denen die beiden folgenden Bedingungen erfüllt sind:
(a) n = m = 0 und
(b) R₁, R₂, R₃ bedeuten eine C₁₋₄-Alkylgruppe oder R₃ bedeutet Wasserstoff und R₁ und R₂ bilden einen C₅₋₈-Ring, der gegebenenfalls mit einer oder zwei Alkylgruppen substituiert ist;
(2) Verbindungen, bei denen die beiden folgenden Bedingungen erfüllt sind:
(a) n = 1 und R₄ bedeutet eine Alkylgruppe und insbesondere Methyl oder m = 1 und R₅ bedeutet eine Alkylgruppe und insbesondere Methyl und
(b) R₁ und R₂ bedeuten eine C₁₋₄-Alkylgruppe und insbesondere Methyl;
(3) Verbindungen, die ausgewählt sind unter: 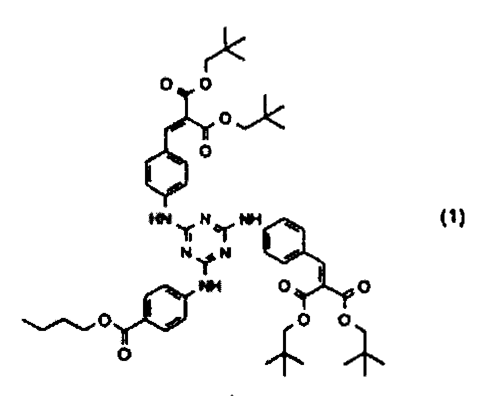 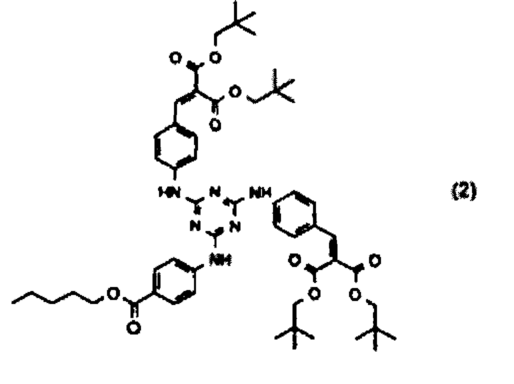 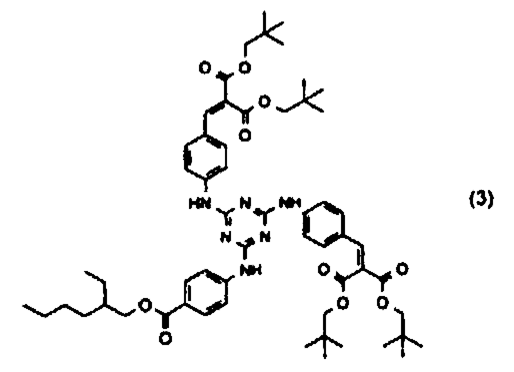 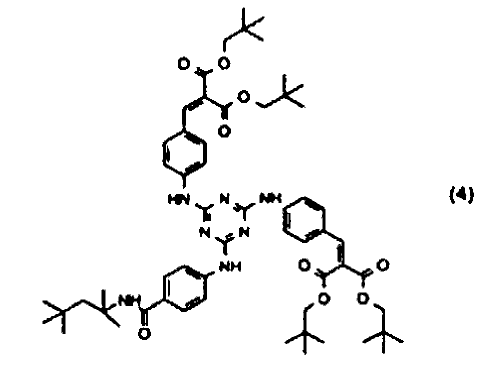 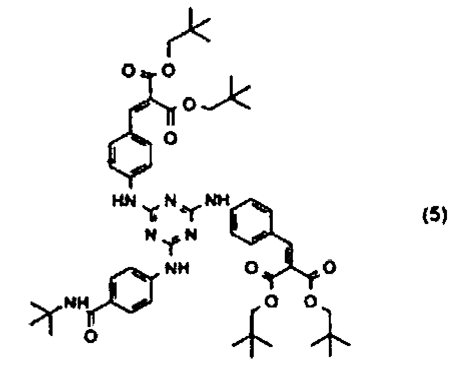 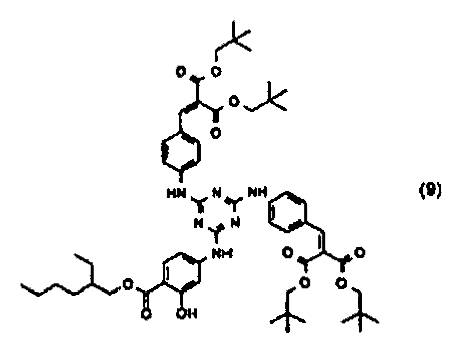 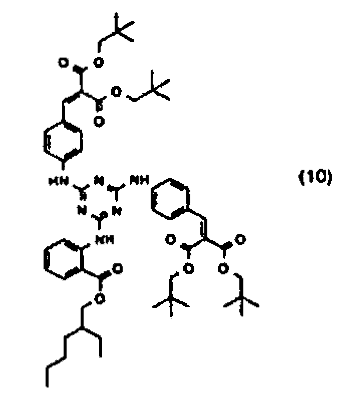
(4) der Verbindung (6) der Formel:

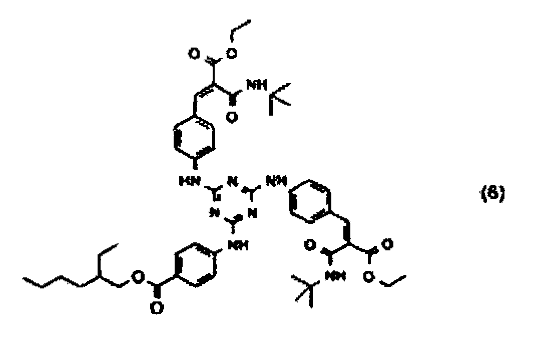

2. Verbindung nach Anspruch 1, **dadurch gekennzeichnet, dass** sie unter den Verbindungen ausgewählt ist, bei denen die beiden folgenden Bedingungen erfüllt sind:
(a) n = m = 0 und
(b) R₁, R₂, R₃ bedeuten Methyl oder R₃ bedeutet Wasserstoff und R₁ und R₂ bedeuten einen Cyclohexylring;

3. Verbindung nach Anspruch 1, **dadurch gekennzeichnet, dass** sie unter den Verbindungen ausgewählt ist, bei denen die beiden folgenden Bedingungen erfüllt sind:
(a) n = 1 und R₄ bedeutet Methyl oder m = 1 und R₅ bedeutet Methyl und
(b) R₁ und R₂ bedeuten Methyl.

4. Verbindung nach einem der Ansprüche 1 bis 3, **dadurch gekennzeichnet, dass** sie unter den Verbindungen der folgenden Formeln (1) bis (10) ausgewählt ist:

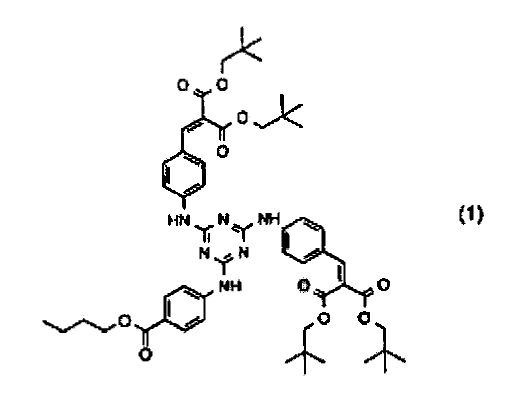

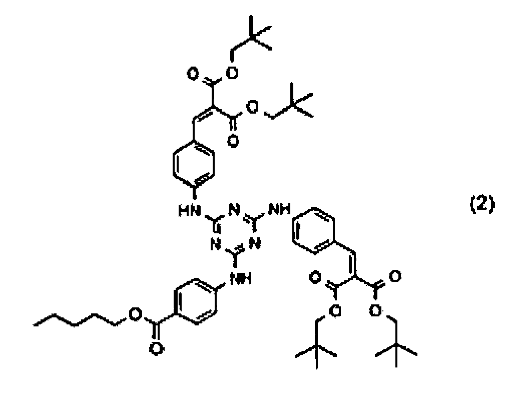

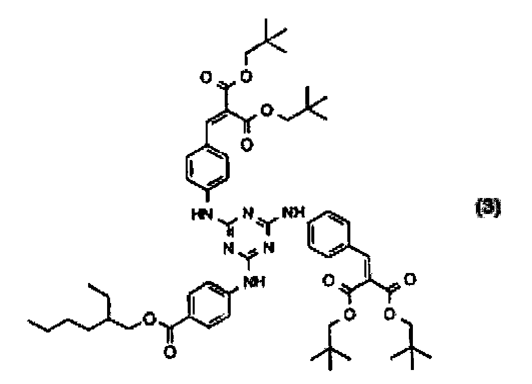

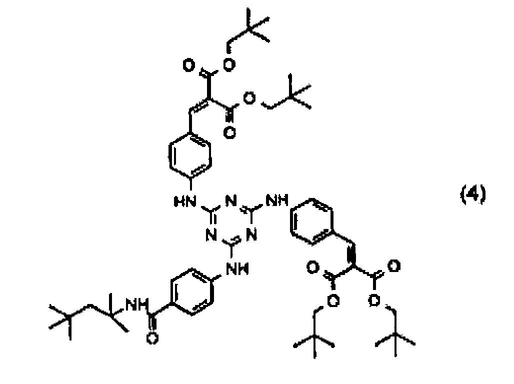

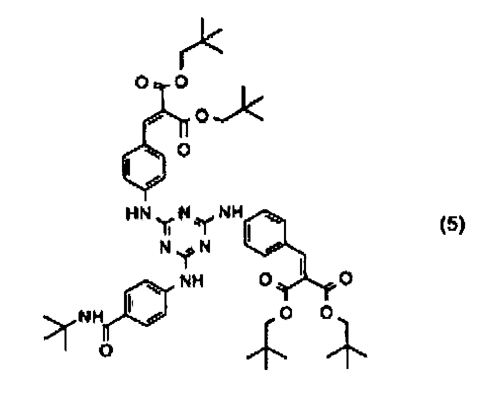

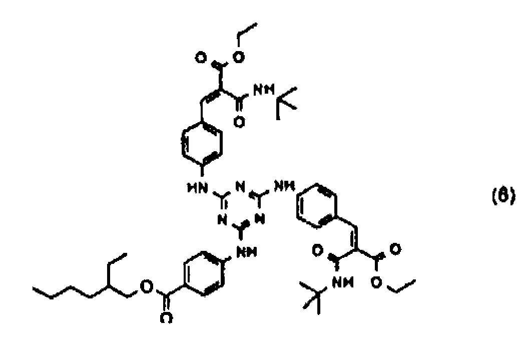

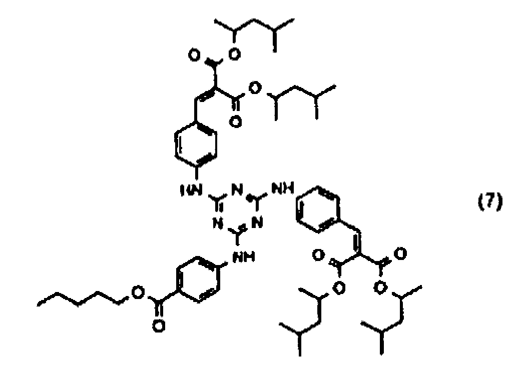

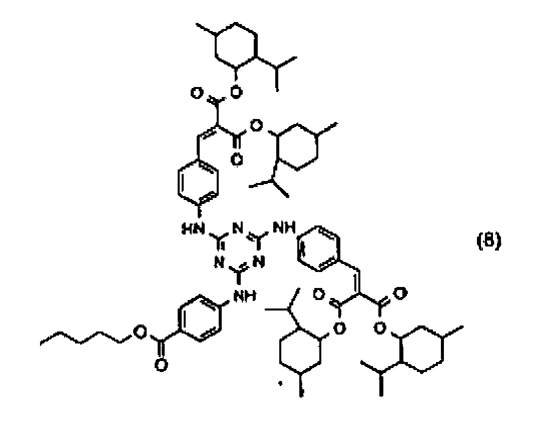

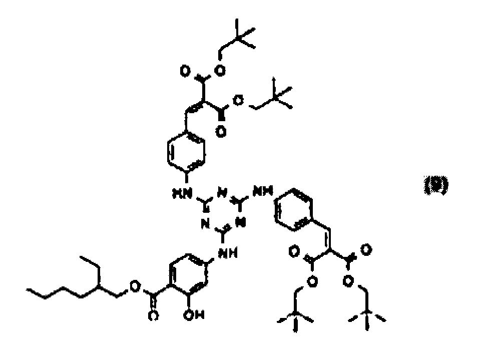

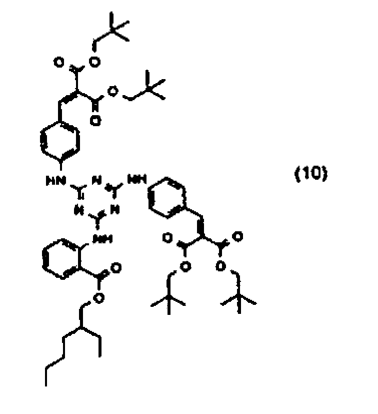

5. Verbindung nach Anspruch 4, **dadurch gekennzeichnet, dass** es sich um das 2,4-Bis-(di-neopentyl-4'-aminobenzalmalonat)-6-(4"-butylaminobenzoat)-s-triazin der Formel (1) handelt.

6. Kosmetische oder dermatologische Zusammensetzung, die in einem physiologisch akzeptablen Medium mindestens eine Verbindung der Formel (I) enthält, wie sie in einem der vorhergehenden Ansprüche definiert ist.

7. Zusammensetzung nach Anspruch 8, wobei die Verbindung(en) der Formel (I) in Mengenanteilen von 0,01 bis 20 Gew.-% und vorzugsweise 0,1 bis 10 Gew.-%, bezogen auf das Gesamtgewicht der Zusammensetzung, enthalten ist (sind).

8. Zusammensetzung nach Anspruch 6 oder 7, **dadurch gekennzeichnet, dass** sie ferner weitere im UV-A- und/oder UV-B-Bereich wirksame Lichtschutzmittel enthält.

9. Zusammensetzung nach einem der Ansprüche 6 bis 8, **dadurch gekennzeichnet, dass** sie ferner mindestens ein Bräunungsmittel und/oder Mittel zur künstlichen Braunfärbung der Haut enthält.

10. Zusammensetzung nach einem der Ansprüche 6 bis 9, **dadurch gekennzeichnet, dass** sie ferner mindestens einen Zusatzstoff enthält, der unter den Fettsubstanzen, organischen Lösemitteln, ionischen oder nichtionischen, hydrophilen oder lipophilen Verdickungsmitteln, beruhigenden Stoffen, Feuchthaltemitteln, Trübungsmitteln, Stabilisierungsmitteln, Emollientien, Siliconen, Schaumverhütungsmitteln, Parfums, Konservierungsmitteln, anionischen, kationischen, nichtionischen, zwitterionischen oder amphoteren grenzflächenaktiven Stoffen, Wirkstoffen, Füllstoffen, Polymeren, Treibmitteln, Alkalisierungsmitteln oder Ansäuerungsmitteln ausgewählt ist.

11. Verwendung einer Zusammensetzung, wie sie in einem der Ansprüche 6 bis 10 definiert ist, für die Herstellung von Produkten zur kosmetischen Behandlung der Haut, der Lippen, der Nägel, der Haare, der Wimpern, der Augenbrauen und/oder der Kopfhaut.

12. Verwendung einer Zusammensetzung, wie sie in einem der Ansprüche 6 bis 10 definiert ist, für die Herstellung von Produkten zur Pflege der Haut, der Lippen, der Nägel, der Haare und/oder der Kopfhaut.

13. Verwendung einer Zusammensetzung, wie sie in einem der Ansprüche 7 bis 10 definiert ist, für die Herstellung von Produkten zum Schminken.

14. Verwendung einer Verbindung der Formel (I), wie sie in einem der Ansprüche 1 bis 5 definiert ist, in einer kosmetischen oder dermatologischen Zusammensetzung als die UV-Strahlung filternder Stoff.

15. Verwendung einer Verbindung der Formel (I), wie sie in einem der Ansprüche 1 bis 5 definiert ist, als Stoff zur Kontrolle der durch die UV-Strahlung verursachten Farbänderung der Haut.
